Europäisches Patentamt

(19) European Patent Office

Office européen des brevets

(11) **EP 0 758 211 B1**

(12) **EUROPÄISCHE PATENTSCHRIFT**

(45) Veröffentlichungstag und Bekanntmachung des Hinweises auf die Patenterteilung:
**09.12.1998 Patentblatt 1998/50**

(21) Anmeldenummer: 95916575.4

(22) Anmeldetag: 26.04.1995

(51) Int. Cl.$^6$: **A61B 5/00**

(86) Internationale Anmeldenummer:
**PCT/DE95/00573**

(87) Internationale Veröffentlichungsnummer:
**WO 95/30368 (16.11.1995 Gazette 1995/49)**

(54) **VERFAHREN UND VORRICHTUNG ZUR ANALYSE VON GLUCOSE IN EINER BIOLOGISCHEN PROBE**

PROCESS AND DEVICE FOR THE ANALYSIS OF GLUCOSE IN A BIOLOGICAL SAMPLE

PROCEDE ET DISPOSITIF D'ANALYSE DE GLUCOSE DANS UN ECHANTILLON BIOLOGIQUE

(84) Benannte Vertragsstaaten:
**AT BE CH DE DK ES FR GB IE IT LI NL SE**

(30) Priorität: **05.05.1994 DE 4415728**
**08.11.1994 DE 4439900**

(43) Veröffentlichungstag der Anmeldung:
**19.02.1997 Patentblatt 1997/08**

(73) Patentinhaber:
**BOEHRINGER MANNHEIM GMBH**
**68305 Mannheim (DE)**

(72) Erfinder:
 • **ESSENPREIS, Matthias**
  **D-82131 Gauting (DE)**

 • **KNUETTEL, Alexander**
  **D-69469 Weinheim (DE)**
 • **BOECKER, Dirk**
  **D-69115 Heidelberg (DE)**

(74) Vertreter:
 **Pfeifer, Hans-Peter, Dr.,**
 **Dr. H.-P. Pfeifer Dr. P. Jany,**
 **Patentanwälte et al**
 **Beiertheimer Allee 19**
 **76137 Karlsruhe (DE)**

(56) Entgegenhaltungen:
 **EP-A- 0 589 191    WO-A-92/19930**
 **US-A- 5 146 091**

Printed by Xerox (UK) Business Services
2.16.6/3.4

## Beschreibung

Die Erfindung betrifft ein Verfahren zur Analyse von Glucose in einer biologischen Probe sowie ein entsprechendes Glucosemeßgerät.

Der Begriff "biologische Probe" bezeichnet eine Körperflüssigkeit oder ein Gewebe eines lebenden Organismus. Biologische Proben sind meist optisch heterogen, d.h. sie enthalten eine Vielzahl von Streuzentren, an denen eingestrahltes Licht gestreut wird. Im Falle von biologischem Gewebe, insbesondere Hautgewebe, werden die Streuzentren von den Zellwänden und anderen in dem Gewebe enthaltenen Bestandteilen gebildet.

Körperflüssigkeiten, insbesondere Blut, sind ebenfalls optisch heterogene biologische Proben, weil sie Partikel enthalten, an denen das eingestrahlte Licht gestreut wird. Auch Milch und andere in der Lebensmittelchemie zu untersuchende Flüssigkeiten enthalten vielfach eine hohe Konzentration von Streuzentren, beispielsweise in Form von emulgierten Fetttröpfchen.

Die Erfindung ist zur Analyse verhältnismäßig stark streuender, d.h. optischer heterogener biologischer Proben geeignet. Dies schließt jedoch nicht aus, daß mit geeigneten Ausführungsformen der Erfindung auch optisch homogene (d.h. wenig oder praktisch gar nicht streuende) Proben analysiert werden können.

Zur qualitativen und quantitativen analytischen Bestimmung von Komponenten solcher biologischen Proben werden im allgemeinen Reagenzien bzw. Reagenziensysteme eingesetzt, die mit der jeweiligen Komponente chemisch reagieren. Die Reaktion führt zu einer physikalisch nachweisbaren Änderung der Reaktionslösung, beispielsweise einer Änderung ihrer Farbe, die als Meßgröße gemessen werden kann. Durch Kalibration mit Standardproben bekannter Konzentration wird eine Korrelation zwischen den bei unterschiedlichen Konzentrationen gemessenen Werten der Meßgröße und der jeweiligen Konzentration bestimmt. Diese Verfahren ermöglichen Analysen mit hoher Genauigkeit und Empfindlichkeit, machen es jedoch erforderlich, eine flüssige Probe, insbesondere eine Blutprobe, zur Analyse dem Körper zu entnehmen ("Invasive Analyse"). Diese Probenentnahme ist unangenehm und schmerzhaft und führt zu einem gewissen Infektionsrisiko.

Dies gilt vor allem, wenn eine Krankheit sehr häufige Analysen erforderlich macht. Das wichtigste Beispiel ist der Diabetes mellitus. Um schwere Folgeerkrankungen und kritische Zustände des Patienten zu vermeiden, ist es bei dieser Krankheit erforderlich, den Glucosegehalt des Blutes sehr häufig oder sogar kontinuierlich zu bestimmen.

Es sind deshalb bereits eine Vielzahl von Verfahren und Vorrichtungen vorgeschlagen worden, um Glucose in Blut, Gewebe oder anderen biologischen Proben in vivo und nicht-invasiv zu bestimmen.

Ein Überblick über physikochemische (reagenzienfreie) Bestimmungen von Glucose in vivo wird gegeben in: J.D. Kruse-Jarres "Physicochemical Determinations of Glucose in vivo", J. Clin. Chem. Clin. Biochem. 26 (1988), 201-208. Als nicht-invasive Verfahren werden dabei unter anderem die Kernspinresonanz (NMR, nuclear magnetic resonance), Elektronenspinresonanz (ESR, electron spin resonance) sowie die Infrarotspektroskopie genannt. Keines dieser Verfahren hat jedoch bis jetzt praktische Bedeutung erlangen können. Es sind große und aufwendige Apparaturen erforderlich, die für die Routineanalytik oder gar die Selbstkontrolle des Patienten (home monitoring) völlig ungeeignet sind.

Die Erfindung bezieht sich auf eine Teilgruppe solcher Verfahren, bei denen Meßlicht von einem Lichtsender durch eine die Probe begrenzende Grenzfläche als Primärlicht in diese eingestrahlt und aus der biologischen Probe durch eine diese begrenzende Grenzfläche austretendes Licht von einem Lichtempfänger detektiert wird, um eine durch die Wechselwirkung mit der biologischen Probe (ohne Verwendung von Reagenzien) veränderliche physikalische Eigenschaft des Lichts zu bestimmen, die mit der Konzentration von Glucose in der biologischen Probe korreliert. Ein solcher Verfahrensschritt wird nachfolgend als "Detektionsschritt" bezeichnet.

Die in einem Detektionsschritt bestimmte (detektierte) mit der Glucosekonzentration korrelierende physikalische Eigenschaft des Lichtes, die man auch als "quantifizierbarer Parameter" (englisch: quantifiable parameter) bezeichnen kann, wird nachfolgend einfachheitshalber als "Meßgröße" bezeichnet. Dieser Begriff darf aber nicht dahingehend verstanden werden, daß ein bestimmter Betrag der Meßgröße in einer entsprechenden Maßeinheit gemessen werden muß.

Da die hier diskutierten Verfahren in der Regel keine Absolutmessung der Glucosekonzentration ermöglichen, ist (ebenso wie bei den gebräuchlichen, auf chemischen Reaktionen basierenden Analyseverfahren) eine Kalibration erforderlich. Üblicherweise wird in mindestens einem Kalibrationsschritt, der meßtechnisch in gleicher Weise wie der Detektionsschritt ausgeführt wird, die Meßgröße an einer biologischen Probe mit bekannter Glucosekonzentration bestimmt. Dabei kann die jeweilige Glucosekonzentration in der Probe mit irgendeinem vorbekannten Verfahren zur Bestimmung der absoluten Konzentration der Glucose ermittelt werden.

In einem Auswerteschritt des Analyseverfahrens wird die Glucosekonzentration aus der Änderung der Meßgröße bei mindestens einem Detektionsschritt im Vergleich zu mindestens einem Kalibrationsschritt ermittelt. Der Auswerteschritt umfaßt einen Auswertealgorithmus, in dem die Glucosekonzentration in vorbestimmter Weise aus den Ergebnissen von mindestens einem Detektionsschritt ermittelt wird.

Die Wellenlängen des Lichts, die für solche Verfahren diskutiert werden, liegen allgemein zwischen etwa 300 nm und mehreren tausend nm, also im Spektralbereich zwischen dem nahen UV- und infrarotem Licht. Der Begriff "Licht" darf nicht als Einschränkung auf den sichtbaren Spektralbereich des Lichtes verstanden werden.

Nahezu alle bekannten Verfahren dieser Art basieren auf den Prinzipien der Spektroskopie. Grundlage ist dabei die Wechselwirkung des eingestrahlten Primärlichtes mit Vibrations- und Rotationszuständen der zu analysierenden Moleküle. Die Meßgröße ist dabei die Lichtintensität I, deren Abnahme durch Absorption in der biologischen Probe in Abhängigkeit von der Wellenlänge L bestimmt wird. Üblicherweise wird die Schwächung des Lichts als Absorption $E(L) = \lg [I(L)/I_0(L)]$ ausgedrückt, wobei I die Intensität des Sekundärlichtes und $I_0$ die Intensität des Primärlichtes bezeichnen.

Die Vibrations- und Rotations-Grundzustände der Glucose befinden sich im IR-Bereich bei Wellenlängen von mehr als 2500 nm. Sie können wegen der starken Absorption des in biologischen Proben stets in hoher Konzentration gegenwärtigen Wassers nicht für die nicht-invasive Analyse von Glucose verwendet werden. Im Bereich des nahen Infrarot (NIR) ist die Absorption des Wassers geringer (sogenanntes "Wasser-Transmissionsfenster"). Die spektrale Analyse von Glucose in diesem Bereich basiert auf der Absorption durch Obertöne (overtones) und Kombinationsschwingungen der Vibrations- und Rotationsgrundzustände des Glucosemoleküls (vgl. vorstehend zitierter Artikel von Kruse-Jarres und EP-A-0 426 358).

Die praktische Realisierung eines nicht-invasiven Glucose-Sensors auf Basis dieser Prinzipien verursacht außerordentlich große Schwierigkeiten. Das Nutzsignal (die Änderung des Absorptionsspektrums in Abhängigkeit von einer Änderung der Glucosekonzentration) ist sehr gering im Vergleich zu Störsignalen, die insbesondere von der spektralen Absorption von Wasser und anderen stark absorbierenden Komponenten resultieren. Außerdem bildet die starke Streuung im Gewebe oder Blut einen großen Störfaktor.

Zur Lösung dieses Problems wurden zahlreiche unterschiedliche Versuche unternommen. Vielfach sollen dabei die Störeinflüsse durch eine geeignete Wahl der Meßwellenlänge in Verbindung mit einer Differenzmessung eliminiert werden. Weit verbreitet ist vor allem die "Zwei-Wellenlängen-Spektroskopie", bei der eine erste Meßwellenlänge so gewählt ist, daß die Glucose möglichst stark absorbiert, während eine zweite Wellenlänge als Referenzwellenlänge so gewählt ist, daß die Lichtabsorption möglichst wenig von der Glucosekonzentration abhängt. Solche oder ähnliche Verfahren sind beispielsweise Gegenstand der EP-A-0 160 768, der WO 93/00856 und des US-Patentes 5,028,787.

In der europäischen Patentschrift 0 074 428 ist ein Verfahren und eine Vorrichtung zur quantitativen Bestimmung von Glucose durch Laser-Lichtstreuung beschrieben. Dabei wird davon ausgegangen, daß die Glucosemoleküle einen durch die Lösung transmittierten Lichtstrahl streuen und daß sich daraus die Glucosekonzentration ableiten läßt. Entsprechend dieser Theorie wird die Raumwinkelverteilung der aus einer Untersuchungsküvette oder einem untersuchten Körperteil austretenden transmittierten Lichtintensität als mit der Glucosekonzentration korrelierende Meßgröße verwendet. Insbesondere wird die Intensität des transmittierten Lichtes in einem Winkelbereich, in dem die Änderung in Abhängigkeit von der Glucosekonzentration möglichst groß ist, gemessen und in Beziehung zu der an dem Zentralstrahl, welcher die Probe in gerader Richtung durchdringt, gemessenen Intensität gesetzt.

Trotz dieser Bemühungen ist es bisher nicht gelungen, einen praktisch funktionsfähigen nicht-invasiven Glucosesensor zur Verfügung zu stellen.

Der Erfindung liegt die Aufgabe zugrunde, ein Verfahren für die analytische Bestimmung von Glucose in einer biologischen Probe zur Verfügung zu stellen, welches reagenzienfrei und nicht-invasiv arbeitet und eine gute Analysegenauigkeit, zum Beispiel fuhr die Beobachtung der Änderung der Analytkonzentration (Verlaufskontrolle) über einen ausreichenden Zeitraum, ermöglicht.

Die Aufgabe wird durch ein Verfahren gemäß Anspruch 1 und eine Vorrichtung gemäß Anspruch 18 gelöst.

Ein wesentliches Element der Erfindung ist die Erkenntnis, daß für die Analyse von Glucose wesentliche Informationen aus der Interferenz des Meßlichtes, welches einen Teil seines Gesamtmeßlichtweges in der Probe zurückgelegt hat, mit einem auf einem definierten Lichtweg, der nicht durch die Probe führt, verlaufenden Referenzlichtstrahl gewonnen werden können. Um ein Interferenzsignal im Sinne der Erfindung zu erzeugen, sind folgende grundlegenden meßtechnischen Voraussetzungen zu beachten.

Interferenz setzt voraus, daß die interferierenden Teilstrahlen kohärent sind. Für besonders wichtige Ausführungsformen der Erfindung ist Licht mit kurzer Kohärenzlänge besonders bevorzugt, wobei insbesondere eine Leuchtdiode oder eine Superlumineszenzdiode als Lichtsender verwendet wird.

Das Meßlicht und das Referenzlicht werden von dem gleichen Lichtsender abgestrahlt und von dem gleichen Lichtempfänger (Detektor) detektiert. Üblicherweise werden bei Interferenzanordnungen optische Koppler verwendet, um das von einem Lichtsender ausgehende Licht in einen Referenzlichtstrahl und in einen Meßlichtstrahl zu teilen und vor dem Lichtempfänger wieder zusammenzuführen.

Der Gesamtmeßlichtweg (bestehend aus dem primärseitigen Meßlichtweg von dem Lichtsender zu der Proben-Grenzfläche, dem von dem Meßlicht in der Probe zurückgelegten Probenlichtweg und dem Sekundärseitigen Meßlichtweg von der Probengrenzfläche, an der das Meßlicht aus der Probe austritt, bis zu dem Lichtempfänger) und der Referenzlichtweg müssen jeweils eine definierte optische Weglänge haben. Eine "definierte optische Weglänge" in diesem Sinne ist Voraussetzung dafür, daß die meßtechnische Erfassung und Auswertung von Interferenzerscheinungen möglich ist. Die Detektion eines Interferenzsignals setzt voraus, daß die beiden miteinander interferierenden Teilstrahlen an dem Detektionsort (der lichtempfindlichen Oberfläche des Lichtempfängers) kohärent sind. Deswegen müssen beide Lichtwege so beschaffen sein, daß die Kohärenz auf dem Weg zu dem Lichtempfänger erhalten bleibt. Dies setzt voraus, daß die von den Photonen zwischen dem Sender und dem Empfänger zurückgelegten Strecken so genau gleich sind, daß die Kohärenz auf dem Lichtweg nicht in einem meßtechnisch störenden Umfang verloren geht. In diesem Sinn ist der Begriff "(interferometrisch) definierte optische Weglänge" zu verstehen.

Die optische Weglänge ist der Weg der Photonen unter Berücksichtigung der Gruppengeschwindigkeit im Medium. In einem homogenen Medium entspricht die optische Weglänge $l_o$ dem Produkt aus dem Brechungsindex n und der geometrischen Lichtweglänge $l_g$ ($l_o = n \cdot l_g$).

Als Interferenzsignal im Sinne der Erfindung ist ein von dem Lichtempfänger erzeugtes elektrisches Signal bzw. ein Signalanteil anzusehen, das bzw. der davon abhängig ist, daß das Meßlicht und das Referenzlicht optisch interferieren. Ein Interferenzsignal wird also nur gemessen, wenn an dem Meßort (der lichtempfindlichen Fläche des Lichtempfängers) Interferenz der beiden Lichtanteile auftritt.

Um in interferometrischen Meßverfahren den auf Interferenz zurückzuführenden Anteil des Detektorsignals möglichst isoliert zu erfassen, ist es üblich, mit einer Modulation zu arbeiten, durch die die optische Weglänge in mindestens einem der Lichtwege (Referenzlichtweg oder Meßlichtweg) moduliert wird. Gebräuchlich ist beispielsweise die Modulation mit Hilfe eines piezoelektrischen Übertragers (piezoelectric transducer, PZT). Die Modulation führt zu einer oszillierenden geringfügigen Veränderung der Lichtweglänge (mit einer Längenänderung, die in der Regel kleiner als die Wellenlänge des Lichtes ist und einer Frequenz, die üblicherweise bei einigen zig Kilohertz liegt). Diese Lichtweglängen-Modulation in Verbindung mit der Interferenz führt zu einer AC-Komponente mit der Modulationsfrequenz im Signal des Lichtempfängers, das mit üblichen frequenzselektiven Meßverfahren (beispielsweise mit dem Lock-In-Prinzip) selektiv verstärkt und gemessen werden kann. "Messen" bedeutet in diesem Zusammenhang die reproduzierbare meßtechnische Bestimmung einer dem Interferenzsignal entsprechenden elektrischen Größe. Eine Absolutmessung im Sinne einer Zuordnung zu einer Maßeinheit ist in der Regel nicht notwendig.

Interferometrische Meßverfahren sind für andere Anwendungszwecke bekannt und vielfach verwendet worden. Dies gilt insbesondere auch für die Interferometrie unter Verwendung von Lichtsendern mit kurzer Kohärenzlänge, die auch als Niederkohärenz-Interferometrie (Low Coherence Interferometry, LCI) bezeichnet wird. Verwiesen sei beispielsweise auf folgende Publikationen:

Danielson et al: "Guided-wave reflectometry with micrometer resolution", Applied Optics, 26 (1987), 2836-2842

Takada et al: "New measurement system for fault location in optical waveguide devices based on an interferometric technique", Applied Optics, 26 (1987), 1603-1606

Schmitt et al: "Measurement of Optical Properties of Biological Tissues by Low-Coherence Reflectometry", Applied Optics, 32 (1993), 6032-6042

WO 92/19930

In diesen Publikationen werden sowohl technische Objekte (Lichtleitfasern für Informationsübertragungssysteme) als auch biologische Gewebe auf ihre optischen Eigenschaften untersucht bzw. in dem letztgenannten Fall in Form eines dreidimensionalen Bildgebungsverfahrens dargestellt. Die Publikationen (vor allem die WO 92/19930) enthalten eine Vielzahl von meßtechnischen Einzelheiten, die auch bei der vorliegenden Erfindung vorteilhaft verwendet werden können und auf die hier vollinhaltlich Bezug genommen wird. Ein Hinweis auf die Möglichkeit der Analyse der Konzentration von Komponenten biologischer Proben, insbesondere der Glucosekonzentration, ist den Publikationen nicht zu entnehmen.

Aus der US-Patentschrift 5146091 ist ein Verfahren bekannt, bei dem die Konzentration von Glucose mittels eines spektroskopischen Verfahrens, insbesondere mittels der Fourier-Transformation-IR-Spektroskopie (FTIR) bestimmt werden soll. Bei diesem Verfahren wird üblicherweise die Länge des Referenzlichtweges variiert. Bei Verwendung einer breitbandig abstrahlenden Lichtquelle kann dadurch mittels einer Fourier-Transformation eine spektroskopische Messung ohne spektrale Zerlegung des Lichts durchgeführt werden. Bei der Literaturstelle wird die Messung am Trommelfell im Ohr vorgenommen, wobei mittels Luftdruck das Trommelfell oszillierend bewegt werden soll, d.h. die Bewegung eines Spiegels im Referenzlichtweg wird durch die Bewegung der Probe selbst (hier des Trommelfells) ersetzt. Ein Hinweis darauf, daß es vorteilhaft sein könnte, die optische Weglänge, die dar Meßlicht innerhalb der Probe zurücklegt, zu messen um unter Berücksichtigung dieser Meßgröße die Glucosekonzentration zu ermitteln, ist der Literaturstelle nicht zu entnehmen. Im Gegenteil wird unterstellt, daß die Probe so dünn ist, daß der von dem Licht innerhalb der Probe Zurückgelegte Lichtweg bei der Auswertung vernachlässigt werden kann.

Das Interferenzsignal kann in dem Auswerteschritt auf unterschiedliche Weise zur Ermittlung der Glucosekonzentration verwendet werden. Insbesondere sind die nachfolgend erläuterten Verfahrensweisen bevorzugt, wobei nähere Erklärungen anhand bevorzugter Ausführungsformen im Zusammenhang mit der Beschreibung der Figuren gegeben werden.

Das erste und nach derzeitigem Kenntnisstand der Erfinder aussichtsreichste dieser Verfahren geht von der Erkenntnis aus, daß der optische Weg von Photonen innerhalb einer biologischen Probe (die man auch als Matrix bezeichnen kann) in einem analytisch nutzbaren Ausmaß von der Glucosekonzentration abhängt. Mit Hilfe des interferometrischen Verfahrens läßt sich mit hoher Genauigkeit eine Meßgröße bestimmen, die der Gruppengeschwindigkeit des Lichts in der Probe oder mit anderen Worten dem Brechungsindex der Probe entspricht. Wie dies geschehen kann, wird anhand der Figuren weiter unten erläutert.

Bei einer zweiten Verfahrensweise wird eine Meßgröße erfaßt, die einer Änderung des Streuquerschnittes streuender Teilchen in der biologischen Probe entspricht. Zu diesem Zweck wird eine Reflexionsanordnung verwendet, bei der aus der Probe reflektiertes Licht in den sekundärseitigen Meßlichtweg eintritt. Geometrisch ausgedrückt befindet sich der primärseitige Meßlichtweg und der sekundärseitige Meßlichtweg in dem gleichen von einer Grenzfläche der Probe definierten Halbraum, wobei in der Reflexions-Interferometrie üblicherweise die gleichen optischen Elemente den primärseitigen und den sekundärseitigen Meßlichtweg bilden, d.h. aus der Probe in den gleichen Meßlichtweg zurückreflektiertes Licht erfaßt wird. Außerdem ist es bei diesem Verfahren erforderlich, einen Lichtsender mit kurzer Kohärenzlänge zu verwenden. Um eine dem Streuquerschnitt entsprechende, für die Glucosekonzentration charakteristische Meßgröße zu bestimmen, wird die Relation der Weglänge des Probenlichtweges im Verhältnis zu der Weglänge des Referenzlichtweges auf unterschiedliche Werte eingestellt. Dies kann durch Veränderung der Länge des Probenlichtweges und/oder des Referenzlichtweges geschehen, wobei die Änderung des Referenzlichtweges konstruktiv einfacher und deswegen bevorzugt ist. Die Relation der optischen Weglängen wird dabei so eingestellt, daß das Interferenzsignal Reflexionen in unterschiedlichen Tiefen der Probe entspricht, wobei die Konzentration der Glucose aus der Abhängigkeit des Interferenzsignals von der Relation der optischen Weglängen (d.h. von der Tiefe der Probe, aus der das Meßlicht reflektiert wurde) ermittelt wird

Die Erfindung wird im folgenden anhand von in den Figuren dargestellten Ausführungsbeispielen näher erläutert, es zeigen:

Fig. 1    ein schematisches Blockdiagramm eines für die Erfindung verwendbaren Reflexions-Interferometers,

Fig. 2    eine graphische Darstellung experimenteller Ergebnisse mit dem Streuquerschnitt als Meßgröße,

Fig. 3    ein schematisches Blockdiagramm eines für die Erfindung verwendbaren Transmissions-Interferometers,

Fig. 4    eine stark schematisierte Querschnittsdarstellung des Auges zur Erläuterung einer Ausführungsform der Erfindung,

Fig. 5    eine zu Fig. 4 analoge Darstellung von einem experimentellen Modell der Vorderkammer des Auges,

Fig. 6a und 6b    zwei Interferogramme eines mit dem Experimentalmodell gemäß Fig. 5 durchgeführten Experimentes,

Fig. 7a und 7b    durch Fouriertransformation erzeugte Phase-Frequenz-Diagramme zu den Meßergebnissen gemäß Fig. 6a und 6b,

Fig. 8    eine graphische Darstellung der Korrelation der experimentell bestimmten Meßgröße (Phase-Slope-Difference) mit der Glucosekonzentration,

Fig. 9    eine graphische Darstellung der Abhängigkeit des inkrementellen Brechungsindexes einer Glucoselösung gegenüber reinem Wasser in Abhängigkeit von der Wellenlänge des Lichts,

Fig. 10    eine graphische Darstellung der Abhängigkeit des gemäß der Erfindung gemessenen Brechungsindexes von der Glucosekonzentration für zwei verschiedene Wellenlängen des Lichts,

Fig. 11    eine graphische Darstellung der Abhängigkeit des differenziellen Brechungsindexes der beiden Wellenlängen gemäß Figur 10 von der Glucosekonzentration.

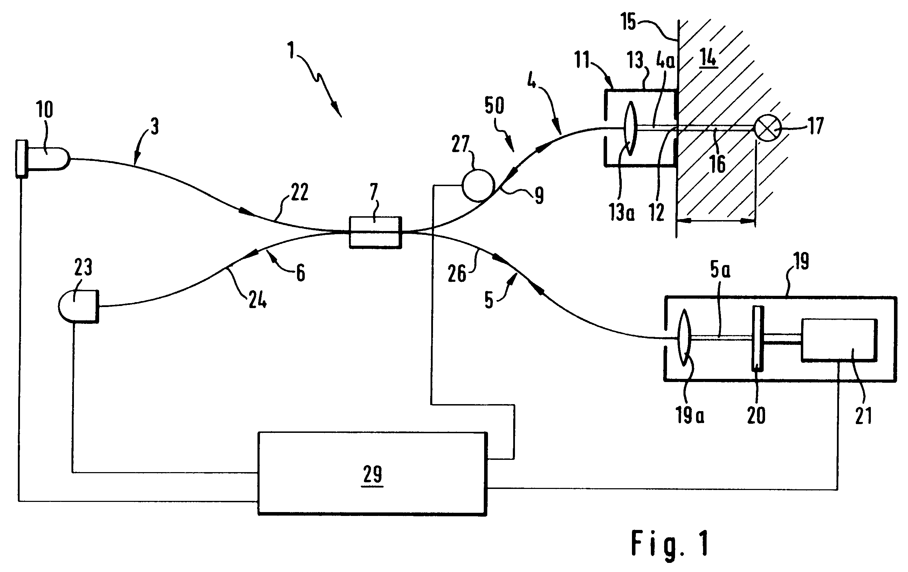

Fig. 1 zeigt den prinzipiellen Aufbau eines erfindungsgemäßen Kurzkohärenz-Reflexions-Interferometers 1 (short coherence reflection interferometer, nachfolgend auch als SCRI bezeichnet). Es hat einen Lichtsenderarm 3, einen Probenarm 4, einen Referenzarm 5 und einen Detektorarm 6, die durch einen Lichtkoppler 7 miteinander verbunden sind.

In der dargestellten bevorzugten Ausführungsform ist das Interferometer 1 faseroptisch aufgebaut, d.h. die Lichtwege des Interferometers werden durch Monomode-Lichtleitfasern (single mode optical fibers) gebildet und der Lichtkoppler ist ein faseroptischer Koppler.

Das von einem Halbleiter-Lichtsender 10, vorzugsweise einer Superlumineszenzdiode, abgestrahlte Meßlicht, welches eine kurze Kohärenzlänge (und zugleich entsprechend große spektrale Bandbreite) hat, wird in einen Eingang des optischen Kopplers 7 eingekoppelt. Über den Probenarm 4 wird das Meßlicht zu insgesamt mit 11 bezeichneten Einstrahlungsmitteln geleitet. Die Einstrahlungsmittel 11 umfassen einen Meßkopf 13 und eine Lichtöffnung 12, durch die das Meßlicht in die Probe 14 eingestrahlt wird. Die Grenzfläche, durch die das Licht in die Probe eindringt, ist mit 15 bezeichnet.

In einem besonders wichtigen Anwendungsfall ist die Probe menschliches Hautgewebe, insbesondere an der Fingerbeere, der Oberbauchdecke, der Lippe, der Zunge, dem inneren Oberarm oder es ist das Gewebe der Skleren, wobei die Gewebeoberfläche die Grenzfläche 15 bildet. Soweit die biologische Probe eine Flüssigkeit (insbesondere Blut) ist, die sich in einem optisch transparenten Gefäß (Küvette) befindet, ist als Grenzfläche der biologischen Probe die Grenzfläche zwischen der Flüssigkeit und der inneren von der Flüssigkeit benetzten Gefäßwand anzusehen. Nachfolgend wird ohne Beschränkung der Allgemeinheit auf Hautgewebe als Probe Bezug genommen.

Das in die Probe auf einem Probenlichtweg 16 eindringende Meßlicht wird von einem symbolisch dargestellten Streuzentrum 17 in Richtung auf den Meßkopf 13 reflektiert. Das dabei in die Lichtöffnung 12 des Meßkopfes 13 einfallende Licht gelangt über den Probenarm 4 zurück zu dem Lichtkoppler 7.

Auf der Ausgangsseite des Lichtkopplers 7 ist auch der Referenzarm 5 angeschlossen, durch den ein Anteil der von der Lichtquelle 10 in den Lichtkoppler 7 eingestrahlten Lichtenergie einer insgesamt mit 19 bezeichneten Reflektoranordnung zugeführt wird. Sie enthält einen in der optischen Achse beweglichen Reflektor 20 (Spiegel), der das auftreffende Licht in die entgegengesetzte Richtung zurückreflektiert. Der Reflektor 20 ist in Richtung der optischen Achse beweglich, wobei im dargestellten Beispielsfall als Bewegungseinrichtung 21 ein Linearantrieb dient.

Das von der Probe 14 und dem Reflektor 20 reflektierte Licht wird in dem Lichtkoppler 7 zusammengeführt und gelangt über den Detektorarm 6 zu einem Lichtempfänger 23. Durch die gleichzeitige Anwesenheit des Meßlichtes und des Referenzlichtes im gleichen Raumelement ist (z.B. an der Detektoroberfläche) die räumliche Kohärenzbedingung erfüllt. Interferenz tritt ein, wenn die beiden in dem Detektorarm 7 transportierten Lichtanteile auch zeitlich kohärent sind.

Der primärseitige Meßlichtweg 22, auf dem das Meßlicht in die Probe 14 eingestrahlt wird, besteht bei der Ausführungsform gemäß Figur 1 also aus dem Lichtsenderarm 3 und dem Probenarm 4, während der sekundärseitige Meßlichtweg aus dem Probenarm 4 und dem Detektorarm 6 besteht. Der insgesamt mit 26 bezeichnete Referenzlichtweg wird von dem Lichtsenderarm 3, dem Referenzarm 5 und dem Detektorarm 6 gebildet. Bei der dargestellten Ausführungsform stimmt also der primärseitige Meßlichtweg 22 und der sekundärseitige Meßlichtweg 24 teilweise (nämlich zwischen dem optischen Koppler 7 und der Grenzfläche 15) überein, so daß beide Lichtwege durch die gleichen optischen Bauelemente verlaufen.

Voraussetzung für eine meßtechnische Nutzung der Interferenz im Sinne der vorliegenden Erfindung ist, daß der von dem primärseitigen Meßlichtweg 22, dem Probenlichtweg 16 und dem sekundärseitigen Meßlichtweg 24 gebildete Gesamtmeßlichtweg und der Referenzlichtweg 26 jeweils eine (interferometrisch) definierte Länge haben. Für den bevorzugten Fall, daß der Lichtsender 10 eine kurze Kohärenzlänge (von vorzugsweise weniger als 50 μm, besonders bevorzugt weniger als 10 μm) hat, ist darüberhinaus Voraussetzung für Interferenz, daß die optischen Weglängen des Gesamtmeßlichtweges und des Referenzlichtweges gleich groß sind. Andernfalls ist die erforderliche zeitliche Kohärenz der Lichtanteile nicht gegeben. In der dargestellten Ausführungsform bedeutet dies, daß der optische Lichtweg von dem optischen Koppler 7 durch den Probenarm 4 bis zu dem reflektierenden Streuzentrum 17 einerseits und von dem optischen Koppler 7 durch den Referenzarm 5 bis zu dem Reflektor 20 andererseits gleich groß sein müssen. Nur wenn diese Bedingung innerhalb von durch die Kohärenzlänge des Lichtsenders 10 definierten Grenzen gegeben ist, kann der Lichtsender 23 ein Interferenzsignal messen.

Zur selektiven Erfassung des Interferenzsignals ist im dargestellten Beispiel ein PZT 27 vorgesehen, durch den einer der nur von dem Meßlicht oder nur von dem Referenzlicht durchlaufenen Teil-Lichtwege, im dargestellten Fall der Probenarm 4, in seiner optischen Weglänge moduliert wird. Der PZT 27, der Lichtsender 10 und der Bewegungsantrieb 21 des Reflektors 20 werden von einer Steuer- und Meßelektronik 29 angesteuert, an der auch das Ausgangssignal des Lichtempfängers 23 anliegt. Durch die Meßschaltung der Steuer- und Meßelektronik 29 wird schmalbandig selektiv nur derjenige Anteil des elektrischen Ausgangssignals des Lichtempfängers 23 erfaßt, der der Modulationsfrequenz des PZT entspricht. Elektronische Meßverfahren, die dies ermöglichen, wie beispielsweise das Lock-in-Verfahren, sind bekannt und müssen hier nicht näher erläutert werden.

Wie erwähnt, sind interferometrische Meßverfahren und insbesondere das Verfahren der Kurzkohärenz-Reflexions-Interferometrie (SCRI) für andere Anwendungszwecke bekannt. Dabei werden zahlreiche Variationen und besondere meßtechnische Maßnahmen verwendet, wie sie beispielsweise in den oben erwähnten Literaturstellen beschrieben werden. Diese können auf Basis der hier gegebenen Informationen auch im Rahmen der vorliegenden Erfindung Verwendung finden. Nachfolgend werden lediglich beispielhaft einige Möglichkeiten von auf die Erfindung abgestimmten besonderen Maßnahmen und Alternativen zu dem, was bisher beschrieben wurde, erörtert.

Der geometrische Querschnitt der Monomode-Lichtleitfasern ist so klein, daß sie Lichtwege mit definierter Weglänge bilden, d.h. unterschiedliche Lichtwege durch unterschiedliche Reflexionen an den Faserwänden vermieden werden. Es ist gebräuchlich und auch im Rahmen der Erfindung vorteilhaft, den dünnen Lichtstrahl vor dem Auftreffen auf die Probe 14 bzw. den Reflexionsspiegel 20 aufzuweiten. Bei der in Fig. 1 dargestellten Ausführungsform sind hierfür lediglich symbolisch angedeutete optische Elemente 13a, 19a in dem Meßkopf 13 bzw. der Reflektoranordnung 19 vorgesehen. Der aufgeweitete Lichtstrahl, der einen Teil des Probenarms 4 bzw. des Referenzarms 5 bildet, ist mit 4a bzw. 5a bezeichnet. Die Einstrahlung und die Detektion an der Grenzfläche 15 der Probe 14 erfolgt jeweils in einem definierten Flächenbereich. Dessen Größe muß unter Berücksichtigung der Signalintensität und der Schärfe der Tiefeninformation optimiert werden. Die Signalintensität spricht eher für einen größeren Durchtrittsbereich des Lichts, während die Effizienz der Kohärenz mit zunehmender Größe des beobachteten Bereiches abnimmt. Vorzugsweise liegt der Durchmesser des Flächenpunktes, durch den das Meßlicht in die Probe 14 eintritt und innerhalb dessen aus der Probe austretendes Licht von der Meßanordnung erfaßt wird, zwischen etwa 0,1 mm und 1 mm.

Statt der dargestellten faseroptischen Anordnung kann grundsätzlich auch eine Freistrahloptik verwendet werden, wobei der optische Koppler als Strahlteiler realisiert ist. Die Faseroptik ermöglicht jedoch eine besonders kompakte und kostengünstige Konstruktion.

Die Modulationstechnik kann in mehrerlei Weise variiert werden. Insbesondere kann der Reflektor 20 selbst zur Modulation verwendet werden, wenn er in oszillierende Schwingungen entsprechend der Modulationsfrequenz versetzt wird. Dies erfordert jedoch eine verhältnismäßig schnelle mechanische Bewegung. Es besteht auch die Möglichkeit, mit mehreren unterschiedlichen Lichtsendern zu arbeiten, die unterschiedliche Wellenlängen oder Wellenlängenbereiche haben. Das Licht unterschiedlicher Lichtquellen kann durch zusätzliche Lichtkoppler in den Meßlichtweg eingekoppelt werden.

Bei der dargestellten Ausführungsform wird die Variation der Relation der Weglänge des Gesamt-Meßlichtweges einerseits und des Referenzlichtweges andererseits durch die axiale Verschiebung des Reflektors 20 in der optischen Achse des Referenzlichtstrahls realisiert, während sich die Probe 14 in einer definierten und konstanten Position relativ zu der Austrittsöffnung 12 des Meßkopfes 13 befindet. Es ist grundsätzlich auch möglich, wenn auch konstruktiv aufwendiger, statt der Länge des Referenzlichtweges 26 die Länge der Meßlichtwege 22,24 zu variieren, wobei beispielsweise der Meßkopf 13 relativ zu einer in einer definierten Position befindlichen Probe 14 verschoben werden kann.

Figur 1 zeigt den prinzipiellen Aufbau einer für die Erfindung geeigneten Meßapparatur. In einer praktischen Realisierung werden sämtliche dargestellten Bauelemente mit Ausnahme der Steuer- und Meßelektronik 29 in einem einzigen kompakten Meßmodul integriert, der an einer definierten Stelle mit gleichmäßigem Druck an die Hautoberfläche angedrückt werden kann. Dabei kann es vorteilhaft sein, die Temperatur an der jeweiligen Meßstelle konstant zu halten oder sie zu messen und bei der Ermittlung der Glucosekonzentration zu berücksichtigen.

Mit dem dargestellten Kurzkohärenz-Reflexionsinterferometer ist es möglich, gezielt Interferenzsignale zu messen, die aus einer definierten Tiefe der Probe 14 reflektiert werden. Dadurch kann die Messung auch auf ein verhältnismäßig tief im Körper liegendes Gewebe, wie beispielsweise die Retina (Augennetzhaut) ausgerichtet werden. Wie erwähnt, ist Voraussetzung für das Auftreten eines Interferenzsignals am Empfänger 23, daß der Lichtweg in dem Referenzarm 4 und der Lichtweg in dem Probenarm 5 einschließlich des Probenlichtwegs 16 die gleiche Länge haben (maximal um den Betrag der Kohärenzlänge voneinander abweichen). Um eine Tiefenabtastung (depth scan) zu ermöglichen, wird das Interferometer so aufgebaut, daß die kürzeste im Betrieb eingestellte optische Weglänge des Referenzarmes 5 etwas kürzer als die optische Weglänge des Probenarms 4 (von dem Lichtkoppler 7 bis zu der Grenzfläche 15) ist. Wird ausgehend von dieser Position der Referenzarm durch Bewegung des Reflektors 20 (in Figur 1 von links nach rechts) verlängert, so tritt durch die an der Grenzfläche 15 stattfindende Fresnel-Reflexion ein starker Signalpeak auf, wenn die genannten optischen Weglängen übereinstimmen. Bei weiterer Verlängerung des Referenzarmes 5 verschiebt sich der Reflexionspunkt 17 in der Probe 14 in Richtung auf größere Tiefen, d.h. der Probenweg 16 nimmt zu, wobei die optische Weglänge des Probenweges 16 der Differenz zwischen den optischen Weglängen des Referenzarms 5 und des Probenarms 4 entspricht.

Ein Interferenzsignal tritt an dem Empfänger 23 dabei nur auf, wenn sich in der entsprechenden Tiefe der Probe 14 ein reflektierendes Streuzentrum 17 befindet. In biologischen Proben ist die Dichte von streuenden Strukturen so hoch, daß für nahezu jede Einstellung der Tiefenabtastung Licht reflektiert wird. Selbstverständlich führt die hohe Dichte der Streuzentren und die Gegenwart absorbierender Substanzen in der biologischen Probe 14 dazu, daß der bei weitem größte Anteil des eingestrahlten Lichtes absorbiert wird oder durch Streuung an irgendwelchen auf dem Lichtweg liegenden Streuzentren verlorengeht. Die Kurzkohärenz-Reflexionsinterferometrie erlaubt es jedoch, selektiv nur solche Photonen zu erfassen, die ungestreut zu dem reflektierenden Streuzentrum 17 und von diesem zurück an die Grenzfläche 15 gelangen. Alle anderen Lichtanteile erfüllen nicht die Kohärenzbedingung und werden deswegen nicht als Interferenzsignal detektiert.

Die analytische Konzentration von Glucose in der Probe läßt sich mit der in Figur 1 dargestellten Meßapparatur mit den bereits grundsätzlich erläuterten Verfahren wie folgt bestimmen.

Zur Bestimmung einer dem Brechungsindex entsprechenden Meßgröße mit einer Reflexionsanordnung gemäß Figur 1 wird vorteilhaft die variable Tiefenabtastung verwendet. Der Betrag der Variation der optischen Weglänge des Referenzlichtweges in Relation zu der optischen Weglänge des Meßlichtweges sollte dabei größer sein als die mittlere freie Weglänge des Lichtes in der Probe. Vorzugsweise beträgt er ein Vielfaches dieser Weglänge (beispielsweise ein oder zwei Millimeter), so daß sich der Reflexionspunkt während der Tiefenabtastung über eine Vielzahl von Streuzentren verschiebt. Das dabei gemessene Interferenzsignal zeigt eine charakteristische Struktur in Abhängigkeit von der Abtasttiefe. Diese Struktur des Interferenzsignals dokumentiert die optische Weglänge zwischen den - die Struktur erzeugenden Streuzentren. Demzufolge wird der Abstand zwischen strukturmerkmalen (beispielsweise Peaks) des Interferenzsignals von einer Änderung des Brechungsindex infolge der Änderung der Glucosekonzentration beeinflußt. Beispielsweise ist also der Abstand zwischen zwei bestimmten Peaks in der erwähnten Interferenzsignal-Struktur (Abhängigkeit der Intensität von der Abtasttiefe) ein Maß für die Glucosekonzentration. Dieser Abstand läßt sich aus der Interferenzsignal-Struktur bestimmen, wobei in der Praxis vorzugsweise nicht ein bestimmter Abstand zwischen zwei bestimmten Peaks verwendet wird, sondern die gesamte Strukturinformation mit Bildanalyseverfahren analysiert und mit geeigneten numerischen Verfahren ausgewertet wird.

Die Tiefenabtastung erfolgt vorzugsweise oszillierend, wobei die Verstellkurve des Reflektors beispielsweise dreiecksförmig, sägezahnförmig oder sinusförmig sein kann. Dabei werden die Daten über eine Vielzahl von Oszillationen gesammelt, um das Signal-/Rauschverhältnis zu verbessern.

Bevorzugt werden dabei Messungen mit mehreren verschiedenen Wellenlängen des Meßlichts verwendet. Da der Brechungsindex von der Wellenlänge abhängt, können durch die Messung bei mehreren Wellenlängen zusätzliche Informationen gewonnen werden. In einem einfachen Beispiel können zur Erhöhung der Meßgenauigkeit zwei Wellenlängen verwendet werden, von denen eine eine starke Abhängigkeit des Brechungsindex von der Glucosekonzentration und die andere eine möglichst geringe Abhängigkeit des Brechungsindex von der Glucosekonzentration hat.

Die Größe der Änderung des optischen Weges, die durch die Änderung der Glucosekonzentration im physiologischen Bereich verursacht wird, läßt sich wie folgt abschätzen. Eine Änderung der Glucosekonzentration um 1 mM entspricht einer Brechungsindexänderung von etwa 0,002 %. Bei einer Gesamtlänge des Probenlichtweges 16 von etwa 2 mm (Eindringtiefe in die Probe 1 mm) wird der optische Weg um ca. 40 nm verschoben. Bei einer Wellenlänge des Meßlichts von 800 nm entspricht dies einer Phasenverschiebung um ca. 18°.

Diese Änderung läßt sich meßtechnisch so gut bestimmen, daß eine für die Zwecke der Überwachung des Glucosespiegels von Diabetikern ausreichende Meßgenauigkeit erzielt wird.

Um bei dem zweiten Verfahren zu Ermittlung der Glucosekonzentration aus dem Interferenzsignal eine Meßgröße zu gewinnen, die dem Streuquerschnitt streuender Teilchen in der biologischen Probe entspricht, wird die Intensität des Interferenzsignals I für unterschiedliche Längen des Probenlichtweges 16, also für unterschiedliche Eindringtiefen x bestimmt. Zu diesem Zweck wird die Länge des Referenzlichtweges 26 durch axiale Verschiebung des Reflektors 20 auf unterschiedliche Werte eingestellt. In der Praxis geschieht dies zweckmäßigerweise auch in diesem Falle oszillierend. Bei unterschiedlichen Einstellungen des Reflektors 20 werden automatisch Meßvorgänge ausgelöst, um die Abhängigkeit I(x) der Intensität I von der Eindringtiefe x in einem gewünschten Eindringtiefen-Bereich zu bestimmen. Im Hinblick auf die Meßgenauigkeit soll die Funktion I(x) über einen möglichst großen Bereich der Eindringtiefe x gemessen werden. Praktisch sind mit vertretbaren Intensitäten nach dem gegenwärtigen Stand der experimentellen Erprobung Eindringtiefen von etwa 2 mm möglich. Je größer der abgetastete Eindringtiefenbereich ist, desto größer wird die abgetastete geometrische Länge und damit die Genauigkeit der Messung. In manchen Anwendungsfällen kann es jedoch auch wünschenswert sein, nur einen kleinen Tiefenbereich zu erfassen, um in einer bestimmten Tiefe unter der Hautoberfläche die Glucosekonzentration bestimmen zu können.

Die Korrelation des Streuquerschnitts mit der Glucosekonzentration kann dadurch erklärt werden, daß der Streuquerschnitt in dem heterogenen System des Gewebes von der Relation der Brechungsindices der Streuzentren zu dem Brechungsindex der Gewebsflüssigkeit abhängig ist. Wenn sich der letztgenannte Brechungsindex infolge einer Änderung der Glucosekonzentration ändert, so ändert sich auch der Streuquerschnitt.

Bei dem vorstehend beschriebenen Meßverfahren kann die Berechnung der Glucosekonzentration aus der gemessenen Meßgröße I(x) über die explizite Berechnung des Streuquerschnittes erfolgen, wie dies in der oben zitierten Publikation von Schmitt et al. beschrieben ist. Für die Praxis ist es jedoch vorzuziehen, ein numerisches Korrelationsverfahren anzuwenden, bei dem die Gesamtheit der gewonnenen Meßdaten durch Kalibration mit (aus konventionellen Messungen bekannten) Glucosekonzentrationen korreliert wird. Gebräuchlich ist zu diesem Zweck beispielsweise das partial least square (PLS) Verfahren.

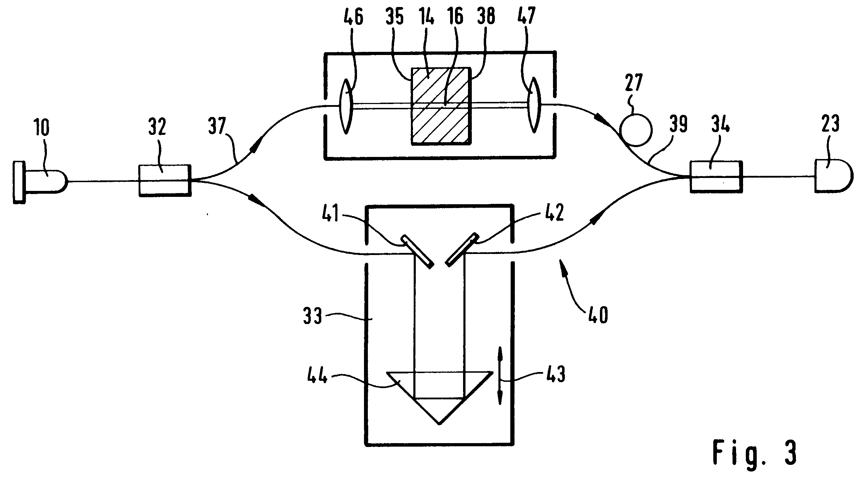

Figur 2 zeigt experimentelle Ergebnisse dieses Verfahrens. Die Messungen wurden an einem Experimentalmodell der Haut vorgenommen, welches aus einer Streulösung von Latexteilchen mit einem Feststoffgehalt von 2,5 Gew. % und einem Durchmesser von 0,2 µm bestand. In dieser Flüssigkeit wurde mit einem SCRI, dessen prinzipieller Aufbau Figur 1 entsprach, eine Tiefenabtastung - wie vorstehend beschrieben - durchgeführt. In Figur 2 ist die gemessene Intensitätsänderung (in dB) in Abhängigkeit von der Eindringtiefe x dargestellt. Die untere Gerade zeigt Meßergebnisse an reinem Wasser ($H_2O$), die obere Meßergebnisse mit einer 400 mMol Glucoselösung (Gl = Glucose). Man sieht, daß sich die Meßgröße I(x) in Abhängigkeit von der Glucosekonzentration deutlich ändert.

Figur 3 zeigt ein Transmissions-Interferometer, bei dem das Licht nicht von der Probe 14 reflektiert wird, sondern diese durchdringt. Eine solche Anordnung ist zur Untersuchung dünner Schichten biologischer Flüssigkeiten oder auch zur in-vivo-Bestimmung der Glucose an entsprechend dünnen Hautfalten (zwischen den Fingern oder am Ohrläppchen) anwendbar. Von einem Lichtsender 10 wird Meßlicht einem ersten Lichtkoppler 32 zugeleitet, der die Lichtenergie in zwei Anteile aufspaltet, von dem ein erster Anteil der Probe 14 und ein zweiter Anteil einer Referenzanordnung 33 mit definierter, vorzugsweise einstellbarer Lichtweglänge zugeführt wird.

Das aus der Probe 14 austretende Meßlicht wird über einen zweiten Lichtkoppler 34 einem Detektor 23 zugeführt. An dem Lichtkoppler 34 erfolgt die Zusammenführung des Meßlichtes mit dem von der Referenzanordnung 33 kommenden Referenzlicht.

Der Weg des Meßlichtes von dem Lichtsender 10 über den ersten Lichtkoppler 32 bis zu der vorderen Grenzfläche 35 der Probe 14, durch die das Meßlicht in diese eindringt, wird als primärseitiger Meßlichtweg 37 bezeichnet. Der Weg des Lichts von der hinteren Grenzfläche 38, durch die das Licht, nachdem es in der Probe 14 den Probenlichtweg 16 zurückgelegt hat, aus der Probe 14 austritt, und dem Lichtempfänger 23 ist der sekundärseitige Meßlichtweg 39. Der Weg des Lichts von dem Lichtsender 10 über den ersten Lichtkoppler 32 und die Referenzanordnung 33 sowie den zweiten Lichtkoppler 34 bis zu dem Lichtempfänger 23 bildet einen Referenzlichtweg 40. Die Länge des Referenzlichtweges 40 ist veränderbar, wobei die Einstellbarkeit beispielsweise, wie in der Figur angedeutet, durch zwei Spiegel 41,42 und ein in Strahlrichtung 43 verschiebbares Prisma 44 realisiert sein kann. Ebenso wie bei Figur 1 ist zur Modulation ein PZT 27 in einen der Lichtwege, im dargestellten Fall in dem sekundärseitigen Meßlichtweg 39, vorgesehen. Optische Elemente 46,47 dienen zur Aufweitung des Strahls vor der Probe 14 und zur Einkopplung des sekundärseitigen Meßlichtes in den Faser-Lichtleiter, der den größten Teil des sekundärseitigen Meßlichtweges 39 bildet.

Bei einer Transmissionsanordnung gemäß Fig. 3 liegt die geometrische Weglänge des Probenlichtweges 16 in der Probe fest. Eine Veränderung des Brechungsindex in der Probe 14 führt zu einer Änderung der optischen Weglänge. Diese läßt sich mit der dargestellten Anordnung unmittelbar messen. Verwendet man eine Lichtquelle 10 mit kurzkohärentem Licht und hat bei einer ersten Glucosekonzentration die Länge des Referenzlichtweges 40 so eingestellt, daß am Detektor 23 ein Interferenzsignal gemessen wird, so hat der Gesamtmeßlichtweg, der aus dem primärseitigen Meßlichtweg 37, dem Probenlichtweg 16 und dem sekundärseitigen Meßlichtweg 39 besteht, die gleiche optische Weglänge wie der Referenzlichtweg 40. Wenn sich danach die Glucosekonzentration und damit die optische Weglänge des Probenlichtweges 16 ändert, ist zur Aufrechterhaltung des Interferenzsignales eine Änderung der Längeneinstellung des Referenzlichtweges erforderlich, die ein unmittelbares Maß für die Glucosekonzentration ist.

Bei Verwendung eines Lichtsenders 10 mit großer Kohärenzlänge, wie beispielsweise eines Lasers, ist die Messung der Änderung der optischen Weglänge des Probenlichtweges 16 über eine Messung der Phasenverschiebung zwischen dem dem PZT 27 zugeführten Modulationssignal und der entsprechenden Modulation des Interferenzsignals ebenfalls möglich.

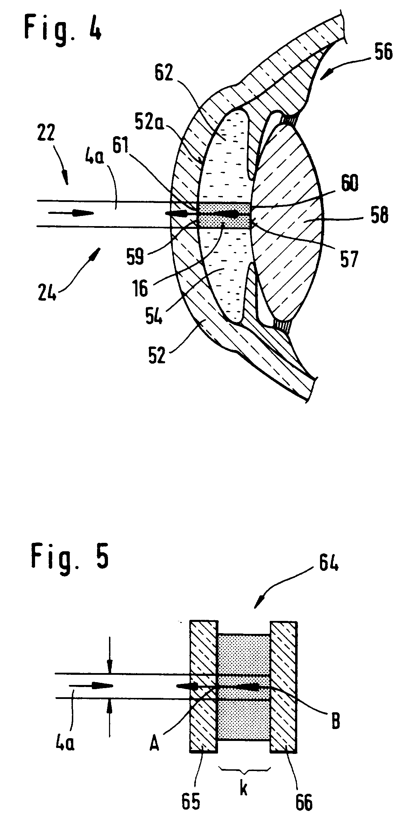

Gemäß einer bevorzugten Ausführungsform der Erfindung, die nachfolgend anhand der Figuren 4 bis 8 näher erläutert wird, schließt die biologische Probe das Kammerwasser in der Vorderkammer des Auges ein. Bevorzugt wird die Messung nur auf die Vorderkammer konzentriert, jedoch ist es auch möglich, weitere (tieferliegende) Teile des Auges in die Messung mit einzubeziehen.

Die Möglichkeit, die Konzentration von Glucose in der Vorderkammer (anterior chamber) des Auges mit Hilfe einer von der Glucosekonzentration abhängigen optischen Eigenschaft des Kammerwassers (aqueous humour) zu bestimmen, wird bereits seit langem diskutiert. In dem 1976 publizierten US-Patent 3,958,560 wird beispielsweise die Möglichkeit beschrieben, einen Lichtstrahl von einer Seite der Vorderkammer unter einem flachen Winkel derartig einzustrahlen, daß die Vorderkammer auf einer Sekante zu der Hornhautkrümmung geradlinig durchläuft und auf der jenseitigen Seite (wiederum unter einem flachen Winkel) austritt ("Vorderkammer-Transmissionsverfahren"). Verfahren, bei denen das Licht von vorne (näherungsweise rechtwinklig zur Hornhautkrümmung) in die Vorderkammer eingestrahlt und aus dieser reflektiertes Licht detektiert wird ("Vorderkammer-Reflexionsverfahren") sind beispielsweise aus den europäischen Patentanmeldungen 0 589 191 und 0 603 658 bekannt. Als Meßgrößen werden in diesen Publikationen die optische Rotation von polarisiertem Licht und die (spektroskopisch gemessene) optische Absorption diskutiert.

Auch im Rahmen der vorliegenden Erfindung besteht grundsätzlich die Möglichkeit, mit einem Vorderkammer-Transmissionsverfahren zu arbeiten, wobei die Meßtechnik gemäß Figur 3 verwendet werden kann. Bevorzugt ist jedoch ein Vorderkammer-Reflexionsverfahren mit Hilfe eines Kurzkohärenz-Reflexionsinterferometers (SCRI), wie es beispielhaft anhand von Figur 1 beschrieben wurde. Bevorzugt wird dabei eine Meßgröße des Lichts gemessen, die dem Brechungsindex des Kammerwassers in der Vorderkammer entspricht. Dabei ist es vorteilhaft, daß das Kammerwasser in der Vorderkammer weitgehend optisch homogen ist und demzufolge das Licht praktisch nicht streut.

Das Prinzip der Meßanordnung ist in Figur 4 dargestellt. Der primärseitige Meßlichtweg 22 ist so gestaltet, daß das Meßlicht von vorne (geometrisch ausgedrückt also unter einem spitzen Winkel zu einer Flächennormalen der Hornhaut 52) in die Vorderkammer 54 des Auges 56 einfällt. Das in dem Auge, insbesondere an der Oberfläche 57 der Augenlinse 58, zurückreflektierte Licht durchsetzt die Hornhaut 52 wiederum unter einem nahezu rechten Winkel (präziser ausgedrückt unter einem spitzen Winkel zu einer Flächennormalen der Hornhaut 52) und fällt in den sekundärseitigen Meßlichtweg 24 ein.

Wie erwähnt wird bei dieser Ausführungsform vorzugsweise ein Kurzkohärenz-Reflexionsinterferometer mit den prinzipiellen Merkmalen der Figur 1 eingesetzt. Wie dort dargestellt ist es insbesondere bevorzugt, daß der primärseitige Meßlichtweg 22 und der sekundärseitige Meßlichtweg 23 in dem zu der Probe führenden Probenarm 4 zusammenfallen (d.h. der Probenarm 4 für beide Meßlichtwege gemeinsam ist). In diesem Fall wird der Lichtwegabschnitt vor dem Auge 56 zweckmäßigerweise von dem aufgeweiteten Lichtstrahl 4a des Probenarmes 4 gebildet. Der Probenlichtweg 16 verläuft bei der Reflexionsmessung am Auge gemäß Figur 4 durch das in der Vorderkammer 54 enthaltene Kammerwasser 62 von einem Lichteintrittsort 59 zu einem Reflexionsort 60 und dann in entgegengesetzter Richtung zu einem Lichtaustrittsort 61. Die Grenzfläche 15 wird durch die dem Augapfel zugewandte innere Oberfläche 52a der Hornhaut 52 gebildet. Bei der dargestellten Ausführungsform mit einem gemeinsamen Probenarm 4 für den primärseitigen Meßlichtweg 22 und den sekundärseitigen Meßlichtweg 24 befindet sich der Eintrittsort 59 und der Austrittsort 61 an der gleichen Stelle der Hornhaut 52. Grundsätzlich (wenn auch weniger bevorzugt) ist es jedoch möglich, mit einem Strahlengang zu arbeiten, bei dem der Probenarm 4 des primärseitigen Meßlichtweges 22 und des sekundärseitigen Meßlichtweges 24 nicht gemeinsam ist, so daß der Eintrittsort und der Austrittsort des Meßlichtes an unterschiedlichen Stellen der Hornhaut 52 liegen und sich auch der Probenlichtweg zwischen dem Eintrittsort und dem Reflexionsort nicht mit dem Probenlichtweg zwischen dem Reflexionsort und dem Austrittsort deckt. Eine derartige Strahlführung ist beispielsweise in der EP 0 603 658 A1 beschrieben.

Das in Figur 5 dargestellte Experimentalmodell ist so gestaltet, daß die wesentlichen Randbedingungen einer Messung an einem menschlichen Auge entsprechen. Das Meßlicht wird von einem Probenarm 4 eines SCRI kommend mit einem Durchmesser von beispielsweise 50 μm in eine Küvette 64 eingestrahlt, die die Vorderkammer 54 simuliert. Die Eintritts-Grenzfläche 15 wird dabei von der inneren Oberfläche des vorderen Küvettenglases 65 gebildet, während sich der Reflexionsort 60 an der inneren Oberfläche des hinteren Küvettenglases 66 befindet. Bei praktischen Versuchen wurde eine Küvettenlänge k von 1 mm gewählt. Hieraus resultiert eine Gesamtlänge des Probenlichtweges 16 ("Roundtrip-Länge") von 2 mm. Im menschlichen Auge beträgt die Dicke der Vorderkammer etwa 3 mm, woraus eine Gesamtlänge des Probenlichtweges 16 von 6 mm resultiert. Insofern wurden die Experimente mit dem in Figur 5 dargestellten Experimentalmodell bei erschwerten Bedingungen durchgeführt.

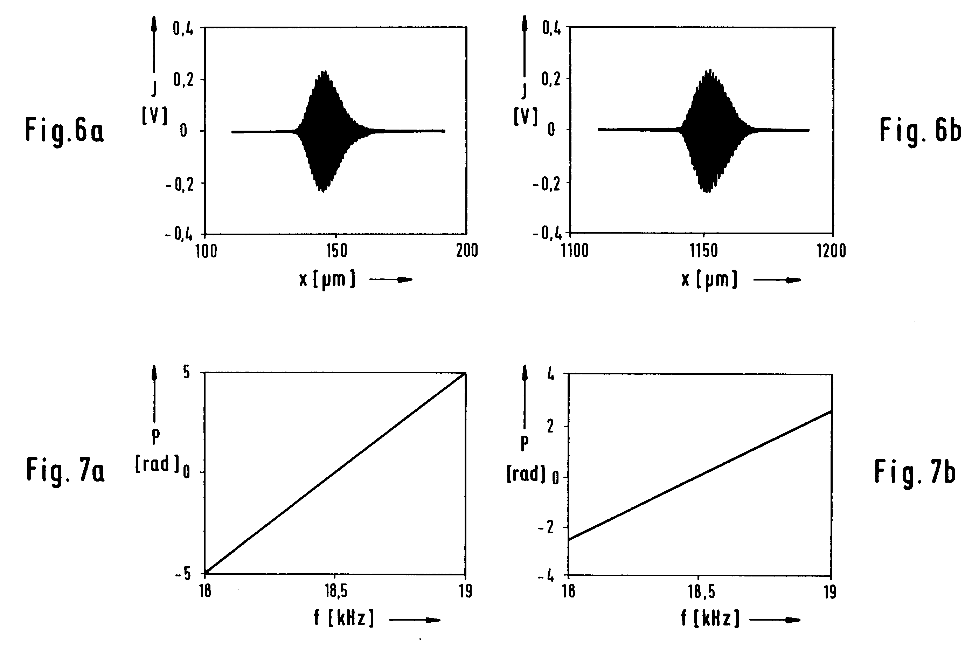

Die in Figur 6 dargestellten Interferogramme wurden an einem Experimentalmodell gemäß Figur 5 mit folgendem Meßbedingungen gemessen.

Der Meßaufbau des SCRI entsprach im wesentlichen Figur 1. Als Lichtquelle wurde eine Superlumineszenzdiode mit einer Leistung von 0,5 mW und einer Zentral-Wellenlänge von 850 nm verwendet. Die Küvette 64 bestand aus Suprasilquarzglas und wurde mit Glucose/Wasser-Lösungen unterschiedlicher Konzentrationen gefüllt. Dabei wurde jeweils von einer Glucosekonzentration von 100 mM ausgegangen und die Konzentration von Messung zu Messung bis auf 1,56 mM halbiert. Die Temperatur in der Küvette war auf eine Temperatur von 22 °C +/- 1°C thermostatisiert. Als Reflektor 20 wurde ein Würfel-Eck-Reflektor (corner cube reflector) eingesetzt, der mit einer Geschwindigkeit von etwa 7,9 mm/s oszillierend bewegt wurde. Die Möglichkeit, durch Verwendung eines oszillierend bewegten Reflektors 20 zugleich eine Tiefenabtastung (depth scan) und eine Modulation der Lichtweglänge zu realisieren, wurde im Zusammenhang mit Figur 1 bereits erläutert. Der dort erwähnte Nachteil einer verhältnismäßig schnellen mechanischen Bewegung des Reflektors 20 wirkt sich bei Messungen am Auge wegen der relativ geringen Gesamt-Abtasttiefe (Dicke der Vorderkammer) relativ wenig aus.

Zur Auswertung des Interferogramms wurde ein Meßdatenverarbeitungsprogramm (data acquisition software) eingesetzt, das es ermöglichte, innerhalb des gesamten TiefenAbtastbereiches zwei Teilbereiche ("Fenster") in der Umgebung der Hauptreflexionen einzustellen, die an der hinteren Fläche des vorderen Küvettenglases 65 und an der vorderen Fläche des hinteren Küvettenglases 66 auftreten. Diese Reflexionspunkte sind in Figur 5 mit A und B bezeichnet. Sie entsprechen bei dem natürlichen Auge dem Eintrittsort 59 (Grenzfläche zwischen Hornhaut und Kammerwasser) und dem Reflexionsort 60 (Grenzfläche zwischen Kammerwasser und Linse).

Bei dem Experimentalmodell gemäß Figur 5 beträgt der Abstand zwischen diesen beiden intensitätsstärksten Reflexionen 1 mm. Entsprechend treten in den beiden in Figur 6 dargestellten Fenstern der Tiefenabtastung (einer Längenskala mit willkürlichem Nullpunkt) Interferogramme bei etwa 150 μm und etwa 1150

$\mu$m, also in einem Abstand von 1000 $\mu$m auf.

Aus den Interferogrammen gemäß Figur 6 läßt sich eine Information über die optische Weglänge des Lichtes zwischen den Reflexionspunkten A und B folgendermaßen bestimmen.

Die Interferogramme gemäß Figur 6 werden digitalisiert und mit einem DSP (Digital Signal Processing)-Prozessor einer schnellen Fouriertransformation (fast fourier transformation, FFT) unterzogen. Die Moduli der transformierten Ergebnisse werden verworfen. Nur die Phasenergebnisse werden weiter verarbeitet. Gemäß der Publikation

W.L.Danielson et al.: "Absolute optical ranging using low coherence interferometry", Appl.Opt., 30, 2975 (1991)

kann man aus dem sogenannten "Phase Slope" (Steigung der funktionalen Abhängigkeit der Phase von der Frequenz) ein genaues Maß der optischen Weglänge gewinnen, die wiederum das Produkt des Brechungsindex n und der geometrischen Weglänge ist.

Figur 7 zeigt die Abhängigkeit der mittels FFT aus den beiden Interferogrammen der Figur 6 ermittelten Phase (gemessen in rad) in Abhängigkeit von der auf der Abszisse aufgetragenen Frequenz (gemessen in kHz). Die Steigung dieser Geraden ist der Phase Slope PS. Gemäß der Publikation von Danielson ergibt sich der Brechungsindex unmittelbar aus der Differenz DPS (Difference of Phase Slopes) der beiden PS-Werte an den Reflexionspunkten A und B gemäß:

$$n = v/\pi \cdot I_g \cdot DPS \qquad (1)$$

Darin ist v die Geschwindigkeit der Reflektorbewegung und $I_g$ die geometrische Gesamtweglänge (doppelte Entfernung zwischen A und B).

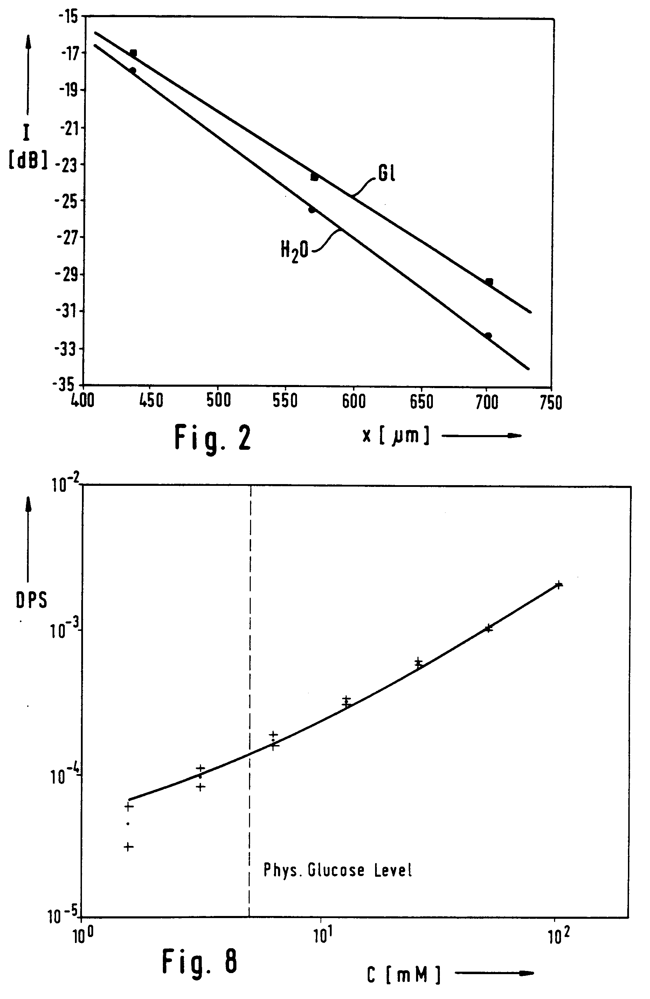

Auf diese Weise läßt sich der Brechungsindex n in der Probe als Maß für die Glucosekonzentration bestimmen. Die am Licht gemessene Meßgröße (d.h. der mit der Glucosekonzentration korrelierende quantifizierbare Parameter des Lichts) ist der DPS-Wert. Die Korrelation der für unterschiedliche Glucosekonzentrationen gemessenen DPS-Werte mit der Glucosekonzentration ist in Figur 8 in einem doppelt logarithmischen Maßstab dargestellt, wobei DPS auf der Ordinate und die Konzentration C auf der Abszisse aufgetragen ist. Die in Figur 8 eingetragenen Punkte bezeichnen Meßwerte für sieben Glucosekonzentrationen, die jeweils mit 32 Abtastvorgängen (scans) innerhalb von zwanzig Sekunden gemessen wurden. Mit den Kreuzen werden die Fehlergrenzen dieser Messung angegeben. Die durchgezogene Linie ist ein "best linear fit" für alle Meßpunkte. Dabei ist der gekrümmte Abschnitt bei niedrigen Werten eine Folge der Tatsache, daß der Fit nicht exakt durch den Nullpunkt geht.

Der Fit entspricht einer Änderung des DPS-Wertes je Einheit der Änderung der Glucosekonzentration von

$2,055 \times 10^{-5}$ rad/Hz pro mM Glucose. Gemäß Gleichung (1) kann daraus eine Änderung des Brechungsindex je Einheit der Konzentration der Glucose von $2,58 \times 10^{-5}$ pro mM berechnet werden. Dies entspricht mit guter Genauigkeit konventionell gemessenen Werten dieser Größe.

Bei Messungen an der Hautoberfläche läßt sich eine weitgehend konstante Länge des primärseitigen und des sekundärseitigen Meßlichtweges dadurch erreichen, daß der Meßkopf 13 (Figur 1) mit gleichmäßigem Druck gegen die Haut gedrückt wird. Dies wird am Auge als unangenehm empfunden. Deswegen wird hier vorzugsweise kontaktlos gearbeitet, d.h. die Einstrahlungsmittel, mit denen das Meßlicht von dem SCRI in das Auge eingestrahlt wird, sind in einem möglichst konstanten Abstand von dem Auge fixiert, wobei dieser konstante Abstand mit für diese Zwecke in der Augenheilkunde gebräuchlichen Mitteln, beispielsweise einer entsprechenden Gesichtsmaske (vgl. EP-0 603 658 A1) gewährleistet wird. Die dabei unvermeidlichen Schwankungen sind jedoch zu groß, um ohne weitere Maßnahmen für die Glucoseanalyse auswertbare Interferogramme messen zu können.

Deswegen werden bevorzugt, wie vorstehend beschrieben, Interferogramme von zwei Reflexionen im Auge gemessen, wobei der Reflexionsort der ersten Reflexion vor der Vorderkammer (auf deren vom Augapfel abgewandten Seite) liegt, während sich der zweite Reflexionsort hinter der Vorderkammer (auf deren dem Augapfel zugewandten Seite) befindet. Vorzugsweise liegt dabei der Reflexionsort der ersten Reflexion an der vom Kammerwasser benetzten Grenzfläche 52a der Hornhaut 52, während der Reflexionsort der zweiten Reflexion an der von dem Kammerwasser benetzten Oberfläche der Augenlinse 58 liegt. Alternativ kann der Reflexionsort der ersten Reflexion auch auf der äusseren Oberfläche der Hornhaut liegen, während der Reflexionsort der zweiten Reflexion alternativ an der Oberfläche der Iris oder auch in der Augenlinse liegen kann. Auch ein tiefer im Auge liegender Reflexionsort, insbesondere an der Oberfläche der Retina, ist grundsätzlich möglich.

Die vorstehenden Ergebnisse zeigen, daß der Brechungsindex und damit die Glucosekonzentration des Kammerwassers der Vorderkammer des Auges mit guter Genauigkeit aus Interferogrammen bestimmt werden kann. Die Nachweisgrenze zu niedrigen Glucosekonzentrationen hängt dabei wesentlich von dem Signal-Rausch-Verhältnis der Messung (SNR) ab. Sie kann durch meßtechnische Optimierung soweit gesenkt werden, daß der gesamte physiologische Konzentrationsbereich der Glucose analysiert werden kann.

Bei Messungen am lebenden Auge ist im Gegensatz zu dem Experimentalmodell gemäß Figur 4 davon auszugehen, daß die geometrische Lichtweglänge $I_g$ nicht langfristig konstant ist, sondern infolge von kleinen Veränderungen der Vorderkammer 54 variiert. In diesem Fall ist es vorteilhaft, wenn der Brechungsindex bzw. die Glucosekonzentration unabhängig von den am lebenden menschlichen Auge vorkommenden Schwankungen der Dicke der Vorderkammer und damit des geometrischen Lichtweges in der Probe bestimmt werden kann. Dies ist mit Messungen bei mindestens zwei unterschiedlichen Licht-Wellenlängen auf Basis der nachfolgenden Überlegungen möglich.

Für eine Lösung von Glucose in Wasser läßt sich DPS für jede Wellenlänge wie folgt ausdrücken:

$$DPS_1 = (n_{w1} + c \cdot dn_{gl1}) l_g \cdot K \qquad (2)$$

$$DPS_2 = (n_{w2} + c \cdot dn_{gl2}) l_g \cdot K$$

Darin bezeichnen die Indices 1 und 2 die beiden Wellenlängen. $n_w$ ist der Brechungsindex des Wassers und $dn_{gl}$ ist die inkrementale Änderung des Brechungsindex je Einheit der Glucosekonzentration. Die Glucosekonzentration ist mit c bezeichnet. K ist eine Konstante.

Wenn man DPS für irgendeine beliebige Glucosekonzentration (beispielsweise für eine Glucosekonzentration von 5 mM) auf Null normiert, kann man die Formeln (2) für relativ (prozentual) kleine Variationen $dl_g$ der geometrischen Weglänge $l_g$ umformen zu:

$$DPS_1 = (dl_g \cdot n_{W1} + c \cdot l_g \cdot dn_{gl1}) \cdot K \qquad (3)$$

$$DPS_2 = (dl_g \cdot n_{w2} + c \cdot l_g \cdot dn_{gl2}) \cdot K$$

Diese Gleichungen lassen sich ohne weiteres nach c auflösen, wobei die unbekannte Änderung $dl_g$ der geometrischen Weglänge $l_g$ herausfällt. Es ergibt sich:

$$c = \frac{DPS_1 - (n_{w1}/n_{w2}) \cdot DPS_2}{K \cdot l_g [dn_{gl1} - (n_{w1}/n_{w2}) dn_{gl2}]} \qquad (4)$$

Die darin noch enthaltene geometrische Weglänge $l_g$ ist der Mittelwert dieser Größe ohne Berücksichtigung der prozentual kleinen Schwankungen $dl_g$. Dieser Mittelwert läßt sich mit ausreichender Genauigkeit aus der relativen Position der beiden Interferogramme (unter Berücksichtigung eines mittleren Brechungsindex) bestimmen. Die Genauigkeit dieser Bestimmung ist besser als die Genauigkeitsanforderungen bei der klinischen Bestimmung der Glucose-Konzentration. Folglich läßt sich die Glucosekonzentration mittels einer Einpunktkalibration bestimmen, bei der der Patient in einem einzigen (zweckmäßigerweise von Zeit zu Zeit wiederholten) Kalibrationsschritt mit einem konventionellen Verfahren die Blut-Glucosekonzentration bestimmt. Grundsätzlich bietet das beschriebene Verfahren sogar die Möglichkeit, den Brechungsindex absolut und damit die Glucosekonzentration ohne Kalibration zu bestimmen.

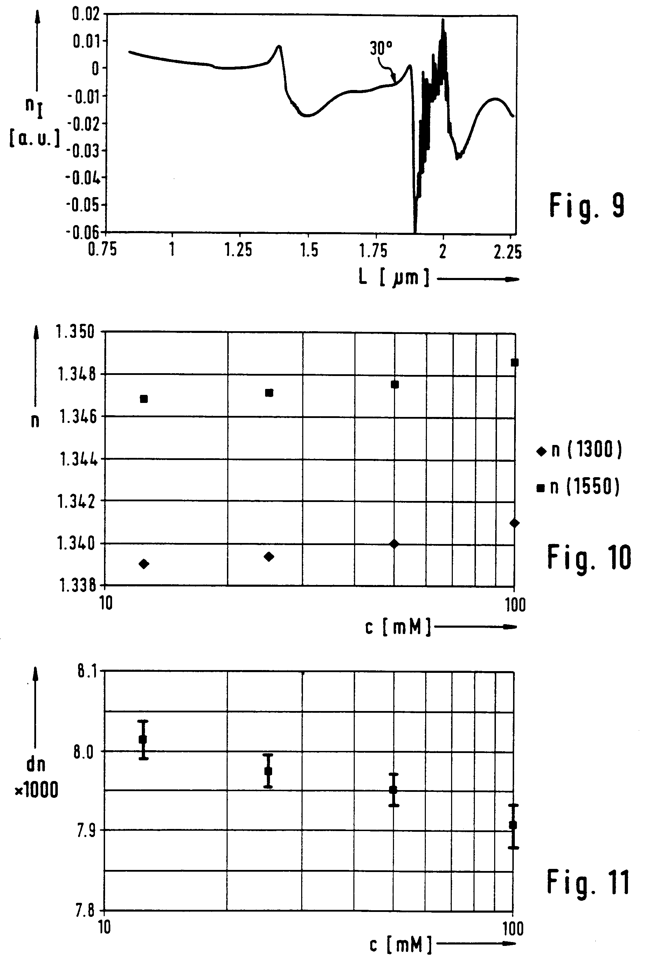

In den Figuren 9 bis 11 sind experimentelle Daten zu der Abhängigkeit des Brechungsindex von der Lichtwellenlänge und der experimentellen Nutzung dieses Effekts im Rahmen der vorliegenden Erfindung dargestellt.

Figur 9 zeigt den inkrementellen Brechungsindex $(n_l)$ einer Glucoselösung gegenüber Wasser, d.h. die Differenz des Brechungsindex der Glucoselösung abzüglich des Brechungsindexes von Wasser. $n_l$ ist in willkürlichen Einheiten (a.u. = arbitrary units) gegen die Wellenlänge L (in μm) aufgetragen. Aus der Darstellung ist zu erkennen, daß der Brechungsindex in bestimmten Wellenlängenbereichen verhältnismäßig stark ändert. Anhand solcher experimentellen Ergebnisse kann der Fachmann ein geeignetes Wellenlängenpaar wählen, bei dem der Unterschied des Brechungsindexes vorzugsweise möglichst groß sein sollte.

Das in Figur 9 erkennbare Maximum bei etwa 1450 nm ist auf die Verdrängung von Wasser durch Glucose im Bereich einer Wasser-Absorptionsbande zurückzuführen. Die leichte Schulter bei etwa 1570 nm geht auf eine Glucose-Absorptionsbande zurück. Dieser Unterschied könnte zusätzlich verwendet werden, um die Einflüsse von Glucose gegenüber anderen Parametern zu trennen.

Auf Basis der in Figur 9 dargestellten Messungen wurden experimentelle Untersuchungen an einem Experimentalmodell der Augenvorderkammer durchgeführt, welches Figur 5 entsprach, wobei jedoch davon ausgegangen wurde, daß die Küvettenlänge k unbekannt ist oder sich in unbekannter Weise ändert.

Die Ergebnisse dieser Untersuchungen sind in den Figuren 10 und 11 dargestellt. Figur 10 zeigt die Änderung des Brechungsindex n mit der Glucosekonzentration für die beiden in der Figur angegebenen Wellenlänge (1300 und 1550 nm) in Abhängigkeit von der in mM angegebenen Glucosekonzentration C. Die dargestellten Brechungsindex-Werte wurden gemäß Formel (1) aus DPS-Werten (wie im Zusammenhang mit Figur 6 und 7 beschrieben) ermittelt. Dabei wurde auf bekannte Tabellenwerte des Brechungsindex von reinem Wasser normiert, so daß die Kenntnis von $l_g$ nicht erforderlich ist.

In Figur 11 ist die Differenz dn der Meßwerte von Figur 10 einschließlich Fehlerbalken gegenüber der Glucosekonzentration aufgetragen. Man sieht eine innerhalb der Fehlergrenze deutlich meßbare Abhängigkeit des differenziellen Brechungsindex dn von der Glucosekonzentration. Die Meßgenauigkeit von dn ist von kleinen Schwankungen der geometrischen Weglänge (d.h. der Küvettenlänge k) weitgehend unabhängig. dn ist deswegen eine geeignete Meßgröße zur Ermittlung der Glucosekonzentration in Fällen, bei denen die geometrische Weglänge des Probenlichtweges nicht konstant ist.

Obwohl das vorstehend erläuterte Meßprinzip (im Gegensatz zur üblichen spektroskopischen Messung) nicht notwendigerweise die Durchführung von Detektionsschritten bei einer Vielzahl von Lichtwellenlängen erfordert, kann es vorteilhaft sein, das Meßlicht mit einer Vielzahl unterschiedlicher Wellenlängen einzustrahlen und das dabei detektierte Licht jeweils in der vorstehend beschriebenen Art und Weise auszuwerten. Es kann auch zweckmäßig sein, das erläuterte Verfahren mit Messungen der optischen Absorption zu kombinieren. Hierdurch kann die Genauigkeit der Messung erhöht werden, wobei insbesondere Störungen und Verfälschungen des Meßergebnisses durch in dem Kammerwasser enthaltene weitere Substanzen wie beispielsweise Ascorbinsäure, Aminosäuren und Milchsäure, die mit der Glucosebestimmung interferieren können, reduziert werden können. Außerdem kann auf diese Weise der mit Temperaturschwankungen in der Vorderkammer des Auges verbundene Meßfehler reduziert werden.

**Patentansprüche**

1. Verfahren zur analytischen Bestimmung der Konzentration von Glucose in einer biologischen Probe, bei welchem in einem Detektionsschritt Meßlicht von einem Lichtsender auf einem primärseitigen Meßlichtweg in die Probe eingestrahlt wird und aus der Probe austretendes Licht auf einem sekundärseitigen Meßlichtweg zu einem Lichtempfänger geleitet und von diesem detektiert wird, um eine durch die Wechselwirkung mit der biologischen Probe veränderliche meßbare physikalische Eigenschaft des Lichtes zu bestimmen, die mit der Konzentration von Glucose in der biologischen Matrix korreliert,
wobei

   ein Teil des von dem Lichtsender abgestrahlten Lichts auf einem Referenzlichtweg mit einer definierten optischen Weglänge dem Lichtempfänger zugeführt wird, der sekundärseitige Meßlichtweg und der Referenzlichtweg vor dem Lichtempfänger derartig zusammengeführt werden, daß das Sekundärlicht und das Referenzlicht miteinander interferieren, der Lichtempfänger ein Interferenzsignal mißt,
   bei dieser Interferenzmessung die optische Weglänge, die das Meßlicht auf einem Probenlichtweg innerhalb der Probe zurücklegt, bestimmt wird,
   der Probenlichtweg ein Teil eines aus dem primärseitigen Meßlichtweg, dem Probenlichtweg und dem sekundärseitigen Meßlichtweg bestehender Gesamtmeßlichtweg mit einer definierten optischen Weglänge ist, und
   die Glucosekonzentration in dem Auswerteschritt unter Berücksichtigung der optischen Weglänge des Probenlichtweges ermittelt wird.

2. Verfahren nach Anspruch 1, **dadurch gekennzeichnet,** daß die optische Weglänge in mindestens einem der Lichtwege mit einem Modulationssignal moduliert und das Interferenzsignal unter Verwendung des Modulationssignals ausgewertet wird.

3. Verfahren nach einem der vorhergehenden Ansprüche, **dadurch gekennzeichnet,** daß das Licht auf dem Referenzlichtweg durch ein reflektierendes optisches Element in die entgegengesetzte Richtung zurückreflektiert wird.

4. Verfahren nach einem der vorhergehenden Ansprüche, **dadurch gekennzeichnet,** daß die optische Weglänge von mindestens einem der Lichtwege veränderlich ist und auf mehrere unterschiedliche Längen eingestellt wird.

5. Verfahren nach einem der vorhergehenden Ansprüche, **dadurch gekennzeichnet,** daß der Lichtsender Licht mit einer kurzen Kohärenzlänge von höchstens 50 µm, bevorzugt höchstens 10 µm erzeugt.

6. Verfahren nach Anspruch 5, **dadurch gekennzeichnet,** daß der primärseitige Meßlichtweg und der sekundärseitige Meßlichtweg in dem gleichen von einer Grenzfläche der Probe definierten Halbraum verlaufen, so daß von der Probe reflektiertes Licht in den sekundärseitigen Meßlichtweg eintritt und daß zur Bestimmung einer Glucosekonzentration mehrere unterschiedliche Relationen der Weglängen des Gesamtmeßlichtweges in Relation zu dem Referenzlichtweg eingestellt werden, bei denen die optische Weglänge des Referenzlichtweges größer als die Summe der optischen Weglängen des primärseitigen Meßlichtweges und des sekundärseitigen Meßlichtweges ist, so daß das Interferenzsignal Reflexionen in unterschiedlichen Tiefen der Probe entspricht.

7. Verfahren nach Anspruch 6, **dadurch gekennzeichnet,** daß die Konzentration der Glucose aus der Abhängigkeit des Interferenzsignals von der eingestellten Relation der optischen Weglängen ermittelt wird.

8. Verfahren nach einem der vorhergehenden Ansprüche, **dadurch gekennzeichnet,** daß die optische Weglänge des Probenlichtweges in dem Auswerteschritt zur Ermittlung des Brechungsindex verwendet und hieraus die Konzentration der Glucose ermittelt wird.

9. Verfahren nach einem der vorhergehenden Ansprüche, **dadurch gekennzeichnet,** daß zur Ermittlung einer Glucosekonzentration mindestens zwei unterschiedliche Meßlicht-Wellenlängen verwendet werden und die Wellenlängenabhängigkeit des Interferenzsignals zur Ermittlung der Glucosekonzentration verwendet wird.

EP 0 758 211 B1

**10.** Verfahren nach einem der vorhergehenden Ansprüche, **dadurch gekennzeichnet**, daß die Temperatur der biologischen Probe gemessen und bei der Ermittlung der Glucosekonzentration berücksichtigt wird.

**11.** Verfahren nach einem der vorhergehenden Ansprüche, **dadurch gekennzeichnet**, daß die biologische Probe eine biologische Flüssigkeit, insbesondere Blut, ist.

**12.** Verfahren nach einem der vorhergehenden Ansprüche, **dadurch gekennzeichnet**, daß die biologische Probe ein biologisches Gewebe, insbesondere an der Fingerbeere, der Oberbauchdecke, dem Nagelbett, der Lippe, der Zunge, dem inneren Oberarm, den Skleren oder der Retina des Menschen ist.

**13.** Verfahren nach einem der vorgehenden Ansprüche, **dadurch gekennzeichnet,** daß die biologische Probe das Kammerwasser in der Vorderkammer des Auges einschließt, wobei das Meßlicht durch die Augenhornhaut in die Vorderkammer des Auges eingestrahlt und aus der Vorderkammer nach Reflexion im Auge durch die Augenhornhaut austretendes Licht detektiert wird, so daß der Probenlichtweg von einem Eintrittsort an der Augenhornhaut innerhalb der Vorderkammer bis zu einem Reflexionsort und von dort zu einem Austrittsort an der Augenhornhaut verläuft.

**14.** Verfahren nach Anspruch 13, **dadurch gekennzeichnet**, daß Interferogramme von zwei Reflexionen im Auge gemessen werden, wobei der Reflexionsort einer ersten Reflexion vor der Vorderkammer des Auges liegt und der Reflexionsort der zweiten Reflexion hinter der Vorderkammer des Auges liegt und die Glucosekonzentration aus den beiden Interferogrammen ermittelt wird.

**15.** Verfahren nach Anspruch 14, **dadurch gekennzeichnet,** daß der Reflexionsort der zweiten Reflexion an der dem Augapfel zugewandten Begrenzung der Vorderkammer des Auges liegt.

**16.** Verfahren nach Anspruch 14, **dadurch gekennzeichnet,** daß der Reflexionsort der zweiten Reflexion an einer Linsenoberfläche der Augenlinse liegt.

**17.** Verfahren nach einem der Ansprüche 14 bis 16, **dadurch gekennzeichnet**, daß der Reflexionsort der ersten Reflexion an der von dem Kammerwasser benetzten Grenzfläche der Augenhornhaut liegt.

**18.** Glucosemeßgerät zur analytischen Bestimmung der Konzentration von Glucose in einer biologischen Probe (14) mit einem Verfahren nach einem der vorhergehenden Ansprüche, umfassend

einen Lichtsender (10) zur Erzeugung von Meßlicht,

Lichteinstrahlungsmittel (11), mit einer Lichtöffnung (12), durch die das Meßlicht durch eine Grenzfläche (15,35) der Probe (14) in die biologische Probe (14) eingestrahlt wird,

einen primärseitigen Meßlichtweg (22,37), der den Lichtsender (10) und die Grenzfläche (15,35) verbindet,

Lichtaufnahmemittel für nach Wechselwirkung mit der Probe (14) an einer Grenzfläche (15,38) aus dieser austretendes Meßlicht und einen sekundärseitigen Meßlichtweg (24,39), der die Grenzfläche (15,38), an der das Meßlicht aus der Probe (14) austritt, mit einem Lichtempfänger verbindet,

einen Referenzlichtweg (26,40), der den Lichtsender (10) und den Lichtempfänger (23) mit einer definierten optischen Weglänge verbindet und

einen in dem sekundärseitigen Meßlichtweg angeordneten optischen Koppler (7) durch den der sekundärseitige Meßlichtweg (24,39) und der Referenzlichtweg (26,40) so zusammengeführt werden, daß sie an der gleichen Stelle auf den Lichtempfänger auftreffen und ein Interferenzsignal erzeugen,

wobei

die Lichteinstrahlungsmittel (11) und die Lichtaufnahmemittel so ausgebildet sind, daß das Meßlicht auf einem Probenlichtweg innerhalb der Probe verläuft, der ein Teil eines aus dem primärseitigen Meßlichtweg, dem Probenlichtweg und dem sekundärseitigen Meßlichtweg bestehender Gesamtmeßlichtweg mit einer definierten optischen Weglänge ist,

die optische Weglänge, die das Meßlicht auf dem Probenlichtweg innerhalb der Probe zurücklegt, bestimmt wird und

die Glucosekonzentration in dem Auswerteschritt unter Berücksichtigung der optischen Weglänge des Probenlichtweges ermittelt wird.

**19.** Glucosemeßgerät nach Anspruch 18, **dadurch gekennzeichnet**, daß das Licht auf mindestens einem Teil der Lichtwege (22,24,37,39;26,40) in Monomode-Lichtleitfasern (9) geführt wird.

**20.** Glucosemeßgerät nach einem der Ansprüche 18 oder 19, **dadurch gekennzeichnet**, daß der Lichtsender (10) ein Dioden-Lichtsender, insbesondere eine Superlumineszenzdiode ist.

**21.** Glucosemeßgerät nach einem der Ansprüche 18 bis 20, **dadurch gekennzeichnet**, daß der Referenzlichtweg (26,40) zur Einstellung seiner optischen Weglänge ein in der optischen Achse verschiebbares reflektierendes optisches Element (20,44) aufweist.

## Claims

1. Method to analytically determine the concentration of glucose in a biological sample,
   wherein, in a detection step, measuring light from a light source is guided along a primary-side measuring light path to be irradiated into the sample, and light emerging from the sample is guided along a secondary-side measuring light path to a photodetector detecting it in order to determine a measurable physical property of light which is variable by the interaction of the light with the biological sample and which correlates with the glucose concentration in the biological matrix,
   wherein

   a portion of the light emitted by the light source is guided along a reference light path of defined optic length to the photodetector, the secondary-side measuring light path and the reference light path are combined before the photodetector in such manner that the secondary light and the reference light interfere with each other, the photodetector detects an interference signal,
   the optical light path length which is travelled by the measuring light on a sample light path inside the sample is determined by this interference measurement,
   the sample light path is a part of a total measuring light path consisting of the primary-side measuring light path, the sample light path and the secondary-side measuring light path, and the glucose concentration is determined in the evaluation step taking into account the optical path length of the sample light path.

2. Method according to claim 1, characterized in that the optical path length of at least one of the light paths is modulated by a modulating signal and the interference signal is evaluated using the modulation signal.

3. Method according to any one of the preceding claims, characterized in that the light on the reference light path is reflected by a light reflecting optical component into the opposite direction.

4. Method according to any one of the preceding claims, characterized in that the optical path length of at least one of the light paths is variable and is set to a plurality of different lengths.

5. Method according to any one of the preceding claims, characterized in that the light source emits light of a short length of coherence of at most 50 μm, preferably at most 10 μm.

6. Method according to claim 5, characterized in that the primary-side measuring light path and the secondary-side measuring light path run in the same half-space defined by a boundary surface of the sample, whereby light reflected by the sample enters the secondary-side measuring light path and in that, in order to determine a glucose concentration, a plurality of different relations of the path lengths of the total measuring light path in relation to the reference light path are set, where the optical path length of the reference light path is larger than the sum of the optical path lengths of the primary-side measuring light path and the secondary-side measuring light path, whereby the interference signal corresponds to reflections at different depths in the sample.

7. Method according to claim 6, characterized in that the glucose concentration is determined from the dependency of the interference signal on the set relation of the optical path lengths.

8. Method according to any one of the preceding claims, characterized in that the optical path length of the sample light path is used in the evaluation step to determine the light refraction index and in that the glucose concentration is derived therefrom.

9. Method according to any one of the preceding claims, characterized in that for the determination of a glucose concentration at least two different measuring-light wavelengths are used and the wavelength-dependency of the interference signal is used for the determination of the glucose concentration.

10. Method according to any one of the preceding claims, characterized in that the temperature of the biological sample is measured and taken into account for the determination of the glucose concentration.

11. Method according to any one of the preceding claims, characterized in that the biological sample is a biological fluid, in particular blood.

12. Method according to any one of the preceding claims, characterized in that the biological sample is a biological tissue, in particular at the finger pad, the upper aodominal wall, the nail bed, lip, tongue and inner upper arm or the tissue of the sclera or retina of a human.

13. Method according to any one of the preceding claims, characterized in that the biological sample comprises the aqueous humor of the anterior chamber of the eye, wherein the measuring light is irradiated through the eye cornea into the anterior chamber and light emerging from the anterior chamber through the cornea after reflection in the eye is detected, whereby the sample light path runs from an irradiation site at the cornea through the anterior chamber up to a reflection site and from there to an exit site at the cornea.

14. Method according to claim 13, characterized in that interferograms of two reflections in the eye are measured, the site of a first reflection being in front of the anterior chamber and the site of the second reflection being located behind the anterior chamber and the glucose concentration is determined from the two interferograms.

15. Method according to claim 14, characterized in that the site of the second reflection is located at the boundary of the anterior chamber facing the eye ball.

16. Method according to claim 14, characterized in that the site of the second reflection is located at the lens surface of the eye lens.

17. Method according to one of claims 14 through 16, characterized in that the site of the first reflection is located at the boundary surface of the eye cornea wetted by the aqueous humor.

18. Glucose-measuring apparatus for the analytical determination of the concentration of glucose in a biological sample (14) by means of a method according to any one of the preceding claims, comprising

   a light source (10) for generating measuring light,
   light-irradiation means (11) having a light aperture (12) by means of which the measuring light is irradiated through a boundary surface (15, 35) of the sample (14) into said sample,
   a primary-side measuring light path (22, 37) connecting the light emitter (10) to the boundary surface (15, 35),
   light detection means for detecting light emerging from the sample (14) at a boundary surface (15, 38) after interaction with the sample, and
   a secondary-side measuring light path (24, 39) connecting the boundary surface (15, 38) where the measuring light emerges from the sample (14) to a photodetector,
   a reference light path (26, 40) of defined optical length connecting the light source (10) and the photodetector (23), and
   an optic coupler (7) positioned in the secondary-side measuring light path, by means of which the secondary-side measuring light path (24, 39) and the reference light path (26, 40) are combined in such manner that they are incident at the same point on the photodetector thereby generating an interference signal, wherein
   the light irradiation means (11) and the light detection means are arranged in such a manner that measuring light runs on a sample light path inside the sample, the sample light path being a part of a total measuring light path having a defined optical path length and consisting of the primary-side measuring light path, the sample light path and the secondary-side measuring light path,
   the optical light path length which is travelled by the measuring light inside the sample is determined and
   the glucose concentration is determined in the evaluation step taking into account the optical path length of the sample light path.

19. Glucose measuring apparatus according to claim 18, characterized in that the light is guided at least along a part of the light paths (22, 24, 37, 39; 26, 40) in single mode optical fibers (9).

20. Glucose measuring apparatus according to any one of claims 18 and 19, characterized in that the light source (10) is a light emitting diode, in particular a superluminescent diode.

21. Glucose measuring apparatus according to any one of claims 18 to 20, characterized in that the reference light path (26, 40) comprises a reflecting optical component (20, 44) which is movable along the optical axis for adjusting the optical path length thereof.

**Revendications**

1. Procédé de détermination analytique de la concentration de glucose dans un échantillon biologique, dans lequel, au cours d'une étape de détection, on projette de la lumière de mesure provenant d'un émetteur de lumière sur un trajet de la lumière de mesure du côté primaire dans l'échantillon et on fait passer la lumière sortant de l'échantillon sur un trajet de la lumière de mesure du côté secondaire vers un récepteur de lumière et on détecte la lumière par celui-ci, pour déterminer une caractéristique physique mesurable de la lumière variant par l'interaction avec l'échantillon biologique, qui est en corrélation avec la concentration de glucose dans la matrice biologique,

une partie de la lumière émise par l'émetteur de lumière étant acheminée sur un trajet de lumière de référence avec une longueur de trajet optique définie, au récepteur de lumière, le trajet de la lumière de mesure du côté secondaire et le trajet de la lumière de référence étant combinés de telle manière avant le récepteur de lumière que la lumière secondaire et la lumière de référence interfèrent ensemble, le récepteur de lumière mesurant un signal d'interférence, en déterminant lors de cette mesure d'interférence la longueur de trajet optique que la lumière de mesure parcourt sur le trajet de la lumière d'échantillon à l'intérieur de l'échantillon,

le trajet de la lumière dans l'échantillon étant une partie du trajet de la lumière de mesure total, constitué du trajet de la lumière de mesure du côté primaire, du trajet de la lumière d'échantillon et du trajet de la lumière de mesure du côté secondaire, avec une longueur de trajet optique définie, et

la concentration de glucose étant déterminée au cours d'une étape d'interprétation en considérant la longueur de trajet optique de la lumière d'échantillon.

2. Procédé selon la revendication 1, caractérisé en ce que la longueur de trajet optique est modulée sur au moins un des trajets de la lumière au moyen d'un signal de modulation et en ce que le signal d'interférence est interprété en utilisant le signal de modulation.

3. Procédé selon l'une des revendications précédentes, caractérisé en ce que la lumière sur le trajet de la lumière de référence est réfléchie dans la direction opposée par un élément optique réfléchissant.

4. Procédé selon l'une des revendications précédentes, caractérisé en ce que la longueur de trajet optique d'au moins un des trajets de la lumière est variable et en ce qu'elle peut être réglée à différentes longueurs.

5. Procédé selon l'une des revendications précédentes, caractérisé en ce que l'émetteur de lumière produit une lumière avec une courte longueur de cohérence d'au maximum 50 $\mu$m, de préférence d'au maximum 10 $\mu$m.

6. Procédé selon la revendication 5, caractérisé en ce que le trajet de la lumière de mesure du côté primaire et le trajet de la lumière de mesure du côté secondaire passent par le même semi-espace défini par l'interface de l'échantillon, de telle manière que la lumière réfléchie par l'échantillon entre dans le trajet de la lumière de mesure du côté secondaire et en ce que pour la détermination d'une concentration de glucose on règle plusieurs relations différentes des longueurs de trajet total de la lumière de mesure en relation avec le trajet de la lumière de référence, pour lesquels la longueur de trajet optique du trajet optique de la lumière de référence est supérieure à la somme des longueurs de trajet optique du trajet de la lumière de mesure du côté primaire et du trajet de la lumière de mesure du côté secondaire, de telle façon que le signal d'interférence correspond à des réflexions à des profondeurs différentes de l'échantillon.

7. Procédé selon la revendication 6, caractérisé en ce que la concentration du glucose est déterminée à partir de la dépendance du signal d'interférence de la relation réglée des longueurs de trajet optique.

8. Procédé selon l'une des revendications précédentes, caractérisé en ce que la longueur de trajet optique du trajet de la lumière d'échantillon dans l'étape d'interprétation est utilisée pour la détermination de l'indice de réfraction et en ce qu'on détermine à partir de celui-ci la concentration de glucose.

9. Procédé selon l'une des revendications précédentes, caractérisé en ce qu'on utilise pour la détermination d'une concentration de glucose au moins deux longueurs d'onde de lumière de mesure différentes et en ce qu'on utilise la dépendance de la longueur d'onde du signal d'interférence pour la détermination de la concentration de glucose.

10. Procédé selon l'une des revendications précédentes, caractérisé en ce que la température de l'échantillon biologique est mesurée et considérée lors de la détermination de la concentration de glucose.

11. Procédé selon l'une des revendications précédentes, caractérisé en ce que l'échantillon biologique est un liquide biologique, en particulier du sang.

12. Procédé selon l'une des revendications précédentes, caractérisé en ce que l'échantillon biologique est un tissu biologique, se trouvant en particulier sur la pulpe de la phalangette, la partie supérieure de l'abdomen, le lit unguéal, la lèvre, la langue, la partie intérieure du bras, les sclères ou la rétine de l'homme.

13. Procédé selon l'une des revendications précédentes, caractérisé en ce que l'échantillon biologique comprend l'humeur aqueuse dans la chambre antérieure de l'oeil, la lumière de mesure étant envoyée par la cornée de l'oeil dans la chambre antérieure de l'oeil et est détectée en provenance de la chambre antérieure après réflexion dans l'oeil grâce à la lumière sortant de la cornée de l'oeil, de telle manière que le trajet de la lumière d'échantillon passe d'un endroit d'entrée situé dans la cornée de l'oeil à l'intérieur de la chambre antérieure jusqu'à un endroit de réflexion et de celui-ci jusqu'à un endroit de sortie se trouvant dans la cornée de l'oeil.

14. Procédé selon la revendication 13, caractérisé en ce qu'on mesure des interférogrammes de deux réflexions dans l'oeil, l'endroit de réflexion de la première réflexion se situant avant la chambre antérieure de l'oeil et l'endroit de réflexion de la deuxième réflexion se situant derrière la chambre antérieure de l'oeil et en ce que la concentration du glucose est déterminée à partir des deux interférogrammes.

15. Procédé selon la revendication 14, caractérisé en ce que l'endroit de réflexion de la deuxième réflexion se situe à la limite de la chambre antérieure de l'oeil du côté du globe oculaire.

16. Procédé selon la revendication 14, caractérisé en ce que l'endroit de réflexion de la deuxième réflexion se situe sur une surface du cristallin de l'oeil.

17. Procédé selon l'une des revendications 14 à 16, caractérisé en ce que l'endroit de réflexion de la première réflexion se situe à l'interface de la cornée de l'oeil mouillée par l'humeur aqueuse.

18. Appareil de mesure du glucose en vue de la détermination analytique de la concentration de glucose dans un échantillon biologique (14) au moyen d'un procédé selon l'une des revendications précédentes, comprenant

un émetteur de lumière (10) pour la production de la lumière de mesure,
des moyens d'irradiation de lumière (11), avec une ouverture pour la lumière (12), par laquelle la lumière de mesure est envoyée dans l'échantillon biologique (14) par une interface (15, 35) de l'échantillon (14),
un trajet de lumière de mesure du côté primaire (22, 37), qui relie l'émetteur de lumière (10) et l'interface (15, 35),
des moyens d'acquisition de la lumière pour la lumière de mesure sortant après interaction avec l'échantillon (14) à l'interface (15, 38) et un trajet de lumière de mesure du côté secondaire (24, 39), qui relie l'interface (15, 38), de laquelle sort la lumière de mesure de l'échantillon (14), à un récepteur de lumière,
un trajet de lumière de référence (26, 40), qui relie l'émetteur de lumière (10) et le récepteur de lumière (23) par une longueur de trajet optique définie et
un coupleur optique (7) disposé sur le trajet de la lumière de mesure du côté secondaire grâce auquel le trajet de la lumière de mesure du côté secondaire (24, 39) et le trajet de la lumière de référence (26, 40) sont combinés de telle manière qu'ils parviennent au récepteur de lumière au même endroit et y produisent un signal d'interférence,
les moyens d'irradiation de lumière (11) et les moyens d'acquisition de lumière étant conçus de telle manière que la lumière de mesure passe par un trajet de lumière d'échantillon à l'intérieur de l'échantillon, qui représente une partie du trajet de lumière de mesure total constitué du trajet de la lumière de mesure du côté primaire, du trajet de lumière d'échantillon et du trajet de la lumière de mesure du côté secondaire, avec une longueur de trajet optique définie,
la longueur de trajet optique que la lumière de mesure parcourt sur le trajet de la lumière d'échantillon à l'intérieur de l'échantillon étant déterminée et
la concentration de glucose étant déterminée dans l'étape d'interprétation en considérant la longueur de trajet optique de la lumière d'échantillon.

19. Appareil de mesure du glucose selon la revendication 18, caractérisé en ce que la lumière est passée sur au moins une partie des trajets de la lumière (22, 24, 37, 39; 26, 40) dans des fibres optiques monomodes (9).

20. Appareil de mesure du glucose selon l'une des revendications 18 ou 19, caractérisé en ce que l'émetteur de lumière (10) est un émetteur de lumière à diode, en particulier une diode superluminescente.

21. Appareil de mesure du glucose selon l'une des revendications 18 à 20, caractérisé en ce que le trajet de la lumière de référence (26, 40), en vue du réglage de sa longueur de trajet optique, présente un élément optique (20, 44) réfléchissant, déplaçable dans l'axe optique.

Fig. 1

EP 0 758 211 B1

Fig. 3

EP 0 758 211 B1

Fig. 4

Fig. 5

Fig.6a

Fig. 6b

Fig. 7a

Fig. 7b

EP 0 758 211 B1

EP 0 758 211 B1

Fig. 2

Fig. 8

23

Fig. 9

Fig. 10

Fig. 11